# EUROPÄISCHE PATENTSCHRIFT

(11) **EP 0 614 457 B1**
(45) Veröffentlichungstag und Bekanntmachung des Hinweises auf die Patenterteilung: **21.08.1996**
(21) Anmeldenummer: 92923741.0
(22) Anmeldetag: 19.11.1992
(51) Int. Cl.: C07C 405/00

(54) **LEUKOTRIEN-B 4?-DERIVATIVE, VERFAHREN ZU IHRER HERSTELLUNG UND ARZNEIMITTEL**
LEUKOTRIENE-B 4? DERIVATIVES, PROCESS FOR PREPARING THE SAME AND MEDICAMENTS
DERIVES DE LEUCOTRIENES-B 4?, LEUR PROCEDE DE PREPARATION ET MEDICAMENTS

(30) Priorität: 29.11.1991 DE 4139868; 29.11.1991 DE 4139869
(43) Veröffentlichungstag der Anmeldung: 14.09.1994
(73) Patentinhaber: SCHERING AKTIENGESELLSCHAFT, D-13353 Berlin (DE)
(72) Erfinder: SKUBALLA, Werner, D-1000 Berlin 28 (DE); BUCHMANN, Bernd, D-1000 Berlin 21 (DE); HEINDL, Josef, D-1000 Berlin 38 (DE); FRÖHLICH, Wolfgang, D-1000 Berlin 31 (DE); EKERDT, Roland, D-1000 Berlin 28 (DE); GIESEN, Claudia, D-1000 Berlin 20 (DE)
(86) Internationale Anmeldenummer: EP9202653
(87) Internationale Veröffentlichungsnummer: WO9311105

(56) Entgegenhaltungen:
- EP-A- 0 399 633
- WO-A-92/16504

## Beschreibung

Die Erfindung betrifft neue Leukotrien-B₄-Derivate, Verfahren zu ihrer Herstellung sowie ihre Verwendung als Arzneimittel. Die neuen Verbindungen sind Strukturanaloge von vorbekannten Leukotrien-B₄-Antagonisten, die einen Sechsring als Grundstrukturelement enthalten (DE-A 39 17 597). Leukotrien-B₄ (LTB₄) wurde von B. Samuelsson und Mitarbeiter als Myetabolit der Arachidonsäure entdeckt. Bei der Biosynthese wird durch das Enzym 5-Lipoxygenase zunächst als zentrales Zwischenprodukt das Leukotrien-A₄ gebildet, das dann durch eine spezifische Hydrolase in das LTB₄ umgewandelt wird.

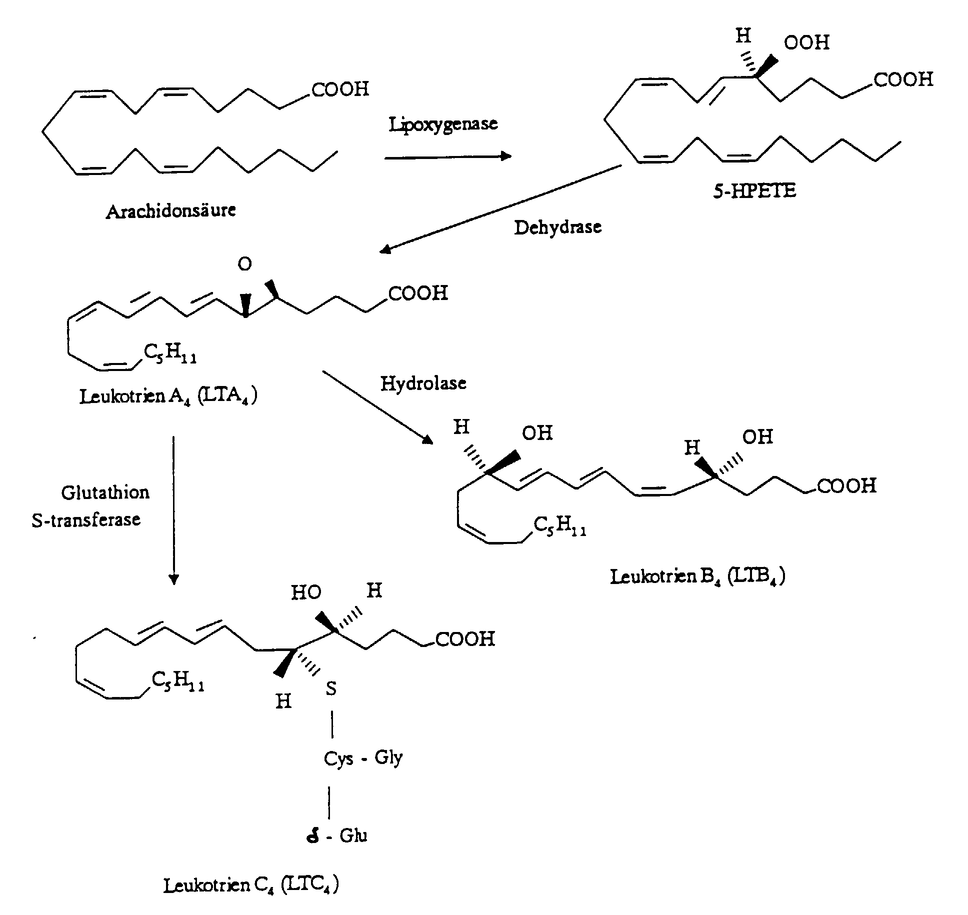

Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukocyten in das erkrankte Gewebe einwandern.
Von LTB₄ weiß man, daß es die Adhäsion von Leukozyten an die Blutgefäßwand verursacht. LTB₄ ist chemotaktisch wirksam, d.h.es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen. Weiterhin sind Leukotriene und LTB₄ insbesondere bei Arthritis, chronischer Lungenerkrankungen (z.B. Asthma), Rhinitis und entzündlichen Darmerkrankungen sowie Reperfusionsschäden verschiedener Organe beteiligt.

Antagonisten gegen LTB₄-Rezeptoren oder -Inhibitoren jener Enzyme, die an der Synthese des LTB₄ beteiligt sind, sollten als spezifische Heilmittel, besonders gegen Krankheiten, die mit Entzündungen und allergischen Reaktionen einhergehen, wirksam sein.

Neben therapeutischen Möglichkeiten, die sich aus einer Antagonisierung der LTB₄-Wirkung mit LTB₄-Analoga ableiten lassen, konnte auch die Nützlichkeit und potentielle Anwendung von Leukotrien B₄-Agonisten zur Behandlung von Pilzerkrankungen der Haut gezeigt werden (H. Katayama, Prostaglandins 34, 797 (1988)).

Die Erfindung betrifft Leukotrien-B₄-Derivate der Formel I, 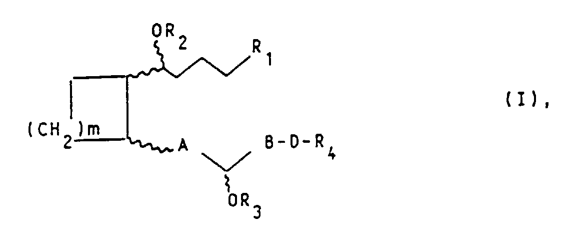 worin
- R¹: CH₂OH, CH₃, CF₃, COOR⁵, CONR⁶R⁷, oder
- R¹: gemeinsam mit R² eine Carbonylgruppe bedeutet,
- R² und R³: gleich oder verschieden H oder ein organischer Säurerest mit 1-15 C-Atomen darstellen,
- R⁴: H, gegebenenfalls einfach oder mehrfach substituierter C₁-C₁₄- Alkyl, C₃-C₁₀-Cycloalkyl, gegebenenfalls unabhängig voneinander einfach oder mehrfach durch Halogen, Phenyl, C₁-C₄-Alkyl, C₁-C₄-Alkoxy, Fluormethyl, Chlormethyl, Trifluormethyl, Carboxyl oder Hydroxy substituierter C₆-C₁₀-Arylrest oder ein 5-6 gliedriger aromatischer heterocyclischer Ring mit wenigstens 1 Heteroatom symbolisieren,
- R⁵: Wasserstoff, C₁-C₁₀ Alkyl, C₃-C₁₀ Cycloalkyl, gegebenenfalls durch 1-3 Halogen, Phenyl, C₁-C₄-Alkyl, C₁-C₄-Alkoxy, Fluormethyl, Chlormethyl, Trifluormethyl. Carboxyl oder Hydroxy substituierter C₆-C₁₀ Arylrest. CH₂-CO-(C₆-C₁₀) Aryl oder ein 5-6 gliedriger Ring mit wenigstens 1 Heteroatom bedeutet,
- A: eine trans.trans-CH=CH-CH=CH, eine -CH₂CH₂-CH=CH- oder eine Tetramethylengruppe,
- B: eine C₁-C₁₀ grad- oder verzweigtkettige Alkylengruppe, die gegebenenfalls durch Fluor substituiert sein kann oder die Gruppe 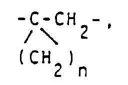 symbolisiert,
- D: eine Direktbindung, Sauerstoff, Schwefel, -C≡C-,-CH=CR⁸, oder gemeinsam mit
- B: auch eine Direktbindung bedeuten können,
- R⁶ und R⁷: gleich oder verschieden sind und H oder C₁-C₄ -Alkyl oder R⁷ H und R⁶ C₁-C₁₅-Alkanoyl oder C₁-C₁₀-Alkansulfonyl darstellen,
- R⁸: H, C₁-C₅ Alkyl, Chlor, Brom bedeutet,
- m: die Ziffern 1 oder 4 symbolisiert und
- n: 3-5 ist sowie, wenn R⁵ Wasserstoff bedeutet, deren Salze mit physiologisch verträglichen Basen und deren Cyclodextrinclathrate.

Die Gruppen OR² und OR³ können α- oder β-ständig sein. Die Formel I umfaßt sowohl Racemate als auch die möglichen reinen Diastereomeren und Enantiomeren.

Als Alkylgruppen R⁵ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Heptyl, Decyl. Die Alkylgruppen R⁵ können gegebenenfalls ein- bis mehrfach substituiert sein durch Halogenatome, Alkoxygruppen, gegebenenfalls substituierte Aryl- bzw. Aroylgruppen mit 6-10 C-Atomen (Bezüglich möglicher Substituenten siehe unter Aryl R⁵), Dialkylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R⁵ sind solche mit 1-4 C-Atomen zu nennen.

Die cycloalkylgruppe R⁵ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als Arylgruppen R⁵ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, eine Fluormethyl-, Trifluormethyl-, -Carboxyl,-Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Als heterocyclische Gruppen R⁵ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R⁶ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenaxyesigäure, Methoxyessigsäure, Ethoxyessigsäure, Hono-, Di- und Trichloressigsäure, Aminoessigsäure. Diethylaminoessigsäure. Piperidinoessigsäure, Morpholinoessigsäure, Milchsäure, Bernsteinsäure, Adipinsäure, benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste und Alkansulfonylreste werden solche mit bis zu 10 Kohlenstoffatomen betrachtet. Als Sulfonsäuren kommen beispielsweise Methansulfonsäure, Ethansulfonsäure, Isopropansulfonsäure, 8-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(B-chlorethyl)-aminosulfansäure, N,N-Diisobutylaminosulfonsäure, N,N-Dibutylaminosulfonsäure, Pyrrolidino-, Piperidino-, Piperazino-, N-Methyl-piperazino- und Morpholinosulfonsäure in Frage.

Als Alkylgruppen R⁴ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl R⁵) substituiert sein können. Beispielsweise genannt seien Methyl-, Ethyl-, Propyl-, Butyl-, Isobutyl-, tert.,-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R⁴ Halogen-substituiert, kommen als Halogene Fluor, Chlor und Brom in Frage.

Als Beispiel für Halogen-substituierte Alkylgruppen R⁴ kommen Alkyle mit terminalen Trifluormethylgruppen in Betracht.

Die Cycloalkylgruppe R⁴ kann im Ring 3-10. vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R⁴ kommen beispielsweise in Betracht: Phenyl, 1-Naphthyl und 2-Naphthyl, die jeweils substituiert sein können durch 1-3 Halogenatome (Fl, Cl, Br), eine Phenylgruppe. 1-3 Alkylgruppen mit jeweils 1-4 C-Atomen eine Chlormethyl-, Fluormethyl-, Trifluormethyl-, Carboxyl-, C₁-C₄Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische aromatische Gruppen R⁴ kommen 5- und 6-gliedrige Heterocyclen in Frage, die wenigstens 1 Heteroatom, vorzugsweise stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe 8 kommen geradkettige oder verzweigtkettige, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1.2-Difluorethylen, 1-Fluorethylen, 1-Methyltetramethylen, 1-Methyl-tri-methylen, 1-Methylen-ethylen, 1-Methylen-tetramethylen.
Die Alkylengruppe 8 kann weiterhin die Gruppe 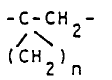 darstellen, wobei n=3-5, bevorzugt 4-5 bedeutet.

Als Säurereste R² und R³ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt.

Beispielsweise seien folgende Carbonsäuren genannt: Ameisensäure. Essigsäure, Propionsäure, Buttersäure, Isobuttersäure, Valeriansäure. Isovaleriansäure, Capronsäure, Önanthsäure. Caprylsäure, Pelargonsäure, Caprinsäure, Undecylsäure. Laurinsäure, Tridecylsäure. Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure. Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-(F, Cl, Br), Trifluormethyl-, Hydroxy-, C₁₋₄-Alkoxy- oder Carboxy-Gruppen substituierte Benzoesäuren, Nikotinsäure. Isonikotinsäure, Furan-2-carbonsäure, Cyclopentylpropionsäure. Als besonders bevorzugte Säurereste R² und R³ werden Acylreste mit bis zu 10 Kohlenstoffatomen betrachtet.

Die Alkylreste R⁶ und R⁷ sind gradkettige oder verzweigte Alkylreste, insbesondere geradkettige wie zum Beispiel Methyl, Ethyl, Propyl, Butyl, Pentyl, Hexyl, besonders bevorzugt Methyl.

R⁸ als C₁₋₅-Alkyl bedeutet geradkettige oder verzweigtkettige Alkylreste wie sie für R⁴ bzw. R⁵ bereits genannt wurden. Bevorzugte Alkylreste R⁸ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Um zu den Cyclodextrinclathraten zu gelangen werden die Verbindungen der Formel I mit α-, β- oder γ-Cyclodextrin umgesetzt. Bevorzugt sind β-Cyclodextrinclathrate.

Bevorzugte Verbindungen der vorliegenden Erfindung sind Verbindungen der Formel I, wobei die Reste die folgende Bedeutung haben:
- R¹: ist CH₂OH, COOR⁵ mit R⁵ in der Bedeutung eines Wasserstoffatoms. eines Alkylrestes mit 1-10 C-Atomen, eines Cycloalkylrestes mit 5-6 C-Atomen, eines gegebenenfalls durch 1-2 Chlor, Brom, Phenyl. C₁₋₄-Alkyl, C₁₋₄-Alkoxy, Chlormethyl, Fluormethyl, Trifluormethyl, Carboxy oder Hydroxy substituierten Phenylrestes
- A: ist eine trans, trans-CH=CH-CH=CH- oder Tetramethylengruppe;
- B: ist eine geradkettige oder verzweigtkettige gesättigte oder unge-Alkylengruppe mit bis zu 10 C-Atomen, die gegebenenfalls durch Fluor substituiert sein kann. oder die Gruppe 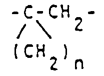 mit n=3-5;
- D: ist eine Direktbindung, Sauerstoff, Schwefel, eine -C≡C-Gruppe oder eine -CH=CR⁸-Gruppe mit R⁸ als Wasserstoff, C₁₋₅-Alkyl, Chlor oder Brom;
- B und D: sind gemeinsam eine Direktbindung;
- R² und R³: sind gleich oder verschieden und bedeuten Wasserstoff oder einen organischen Säurerest mit 1-15 C-Atomen;
- R¹ und R²: sind gemeinsam eine Carbonylgruppe;
- R⁴: ist ein Wasserstoffatom, C₁₋₁₀-Alkyl,
Cycloalkyl mit 5-6 C-Atomen , ein gegebenenfalls durch 1-2 Chlor, Brom, Phenyl, C₁₋₄-Alkyl, C₁₋₄-Alkoxy, Chlormethyl, Fluormethyl, Trifluormethyl. Carboxy oder Hydroxy substituierter Phenylrest und falls
- R⁵: ein Wasserstoffatom bedeutet, deren Salze mit physiologisch verträglichen Basen und deren Cyclodextrinclathrate.

Besonders bevorzugte Verbindungen der vorliegenden Erfindung sind Verbindungen der Formel I, wobei die Reste die folgende Bedeutung haben:
- R¹: ist CH₂OH, COOR⁵ mit R⁵ in der Bedeutung eines Wasserstoffatoms, eines Alkylrestes mit 1-4 C-Atomen;
- R² und R³: sind gleich oder verschieden und bedeuten Wasserstoff oder einen organischen Säurerest mit 1-6 C-Atomen;
- R¹ und R²: sind gemeinsam eine Carbonylgruppe;
- R⁴: ist ein Wasserstoffatom oder C₁₋₁₀-Alkyl;
- A: ist eine trans, trans-CH=CH-CH=CH- oder Tetramethylengruppe;
- B: ist eine geradkettige oder verzweigtkettige Alkylengruppe mit bis zu 5 C-Atomen;
- D: ist eine Direktbindung oder eine -C≡C-Gruppe oder eine -CH=CR₈-Gruppe mit R₈ als Wasserstoff oder C₁₋₅-Alkyl;
- B und D: sind gemeinsam eine Direktbindung;
und falls R⁵ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der Formel I, das dadurch gekennzeichnet ist, daß man einen Aldehyd der Formel II 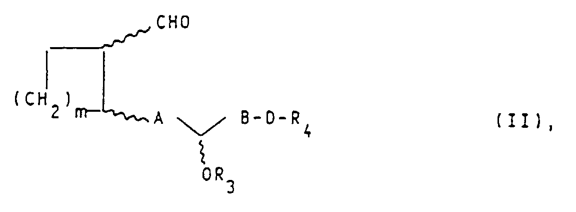 worin m, A, B, D, R³ und R⁴ die oben angegebene Bedeutung haben, gegebenenfalls nach Schutz freier Hydroxygruppen mit einer magnesiumorganischen Verbindung der Formel III,

X-Mg-CH₂-CH₂-CH₂-R⁹ (III),

worin X Chlor, Brom oder Iod und R⁹ -CH₃, CF₃ oder -CH₂OR¹⁰ darstellt, worin R₁₀ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹ =CONHR⁶R⁷) überführt oder eine Carboxylgruppe mit einer physiologisch verträglichen Base in ein Salz überführt.

Als Etherreste R⁹ in der Verbindung der Formel III kommen die dem Fachmann geläufigen Reste in Betracht. Bevorzugt sind leicht abspaltbare Etherreste, wie beispielsweise Dimethyl-tert.-butylsilyl, Trimethylsilyl-. Tribenzylsilyl-. Diphenyl-tert.-butylsilyl-, Tetrahydropyranyl-, Tetrahydrofuranyl- und α-Ethoxyethyl, um nur einige zu nennen.

Die Umsetzung der verbindung der Formel II mit einer metallorganischen Verbindung der Formel III erfolgt in an sich bekannter Weise in einem inerten Lösungsmittel oder Lösungsmittelgemisch, wie zum Beispiel Dioxan, Toluol, Dimethoxyethan oder vorzugsweise Diethylether oder Tetrahydrofuran. Die Reaktion wird bei Temperaturen zwischen -100 °C und 60 °C. vorzugsweise bei -78 °C bis 0 °C durchgeführt.

Die Herstellung der für diese Umsetzung benötigten Verbindung der Formel III erfolgt durch Reaktion des entsprechenden durch eine leicht abspaltbare Ethergruppe geschützten Hydroxyhalogenids und anschließende Umsetzung mit Magnesium.

Die Reduktion zu den Verbindungen der Formel I mit R¹ in der Bedeutung einer CH₂OH-Gruppe wird mit einem für die Reduktion von Estern oder Carbonsäuren geeigneten Reduktionsmittel wie beispielsweise Lithiumaluminiumhydrid. Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R² = H und/oder R³ = H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise NaH, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, OHSO bei Temperaturen über oder unter Raumtemperatur, zum Beispiel zwischen -80 °C bis 100 °C, vorzugsweise bei Raumtemperatur, vorgenommen werden.

Die Oxidation der 1-Hydroxygruppe wird nach den dem Fachmann bekannte Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation (Tetrahedron Letters 1968, 3363 und anschließende Jones-Oxidation. Die Oxidation mit Pyridiniumdichromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C in Aceton als Lösungsmittel ausgeführt.

Die Oxidation mit Platin/Sauerstoff wird bei Temperaturen von 0 °C bis 60 °C, vorzugsweise 20 °C bis 40 °C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen. wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z. B. Salzsäure. durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol. Ethanol. Butanol usw., vorzugsweise Hethanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe -COOR⁵ für R¹, bei welcher R⁵ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8. Seiten 389 - 394 (1954)].

Die Einführung der Estergruppe -COOR⁵ für R¹, bei welcher R⁵ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R⁵ in der Bedeutung eines Wasserstoffs können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe -CONHR₆ mit R₆ in der Bedeutung von Alkanoyl erfolgt nach den dem Fachmann bekannten Methoden. Die Carbonsäuren der Formel I (R₅=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R₆=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe -CONHR₆ besteht in der Umsetzung einer 1-Carbonsäure der Formel I (R₅=H), in der freie Hydroxygruppen gegebenenfalls intermediär geschützt sind, mit Verbindungen der Formel IV,

O = C = N - R₆ (IV),

worin R₆ die oben angegebene Bedeutung hat.

Die Umsetztung der Verbindung der Formel I (R₅=H) mit einem Isocyanat der Formel IV erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, dimethylacetamid, Methylenchlorid, Diethylether, Toluol, bei Temperaturen zwischen -80 °C bis 100 °C, vorzugsweise bei 0 °C bis 30 °C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen im Leukotrien-B₄-Rest so werden diese OH-Gruppen auch zur Reaktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Die Trennung der Enantiomeren und/oder Diastereomeren erfolgt nach den dem Fachmann bekannten Methoden, beispielsweise Hochdruckflüssigchromatographie an optisch aktiven Trägermaterialien.

Die als Ausgangsmaterial dienenden Verbindungen der Formel II mit m in der Bedeutung von 1 können beispielsweise hergestellt werden, indem man in an sich bekannter Weise ein cis- oder trans-Bis-1,2-Hydroxymethylcyclobutan (durch Reduktion aus cis- oder trans- Cyclobutan-1,2-dicarbonsäure erhältlich, siehe z.B. analog A. Padwa et al, J. Org. Chem. 54, 817 (1989); 0. Caamaus et al., Eur.J.Med. Chem. 22, 311 (1987); J. B. Jones et al., J. Am. Chem. Soc., 104, 4659 (1982) in den Monosilylether der Formel Va 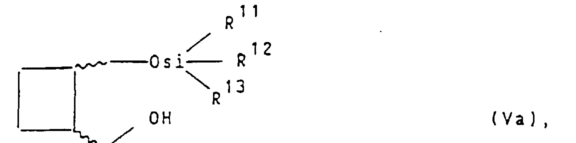 worin
R¹¹, R¹² und R¹⁵ gleich oder verschieden sind und Cₗ-C₄-Alkyl oder Phenyl bedeuten, überführt.

Durch Oxidation z.B. mit Collins-Reagenz oder durch das Swern-Verfahren (Tetrahedron Letters 34, 1651 (1978)) wird der Aldehyd der Formel VIa erhalten, 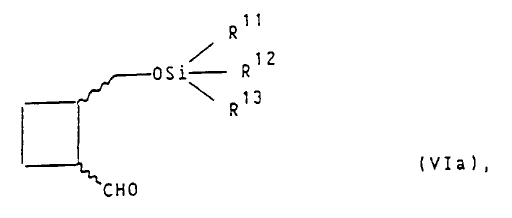 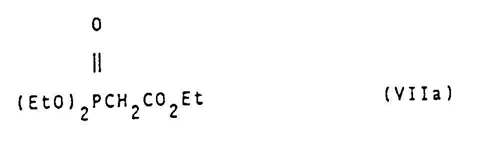 oder 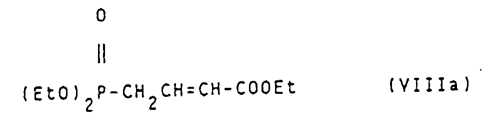 der in einer Wittig-Horner-Olefinierung mit dem Phosphonat der Formel VIIa und einer Base und gegebenenfalls anschließender Hydrierung sowie anschließender Reduktion der Estergruppe, Oxidation des primären Alkohols, nochmaliger Wittig-Horner-Olefinierung mit dem Phospnonat der Formel VIIₐ und gegebenenfails anschließender Hydrierung in den Ester der Formel IXₐ oder einer Wittig-Horner Reaktion des Aldehyds der Formel VI mit einem Phosphonat der Formel VIIIa überführt wird, wobei A die 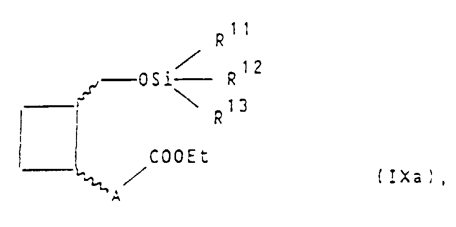 A die oben angegebene Bedeutung besitzt. Als Basen kommen beispielsweise Kaliumtert.-butylat, Diazabicyclononan, Diazabicycloundecan oder Natriumhydrid infrage. Reduktion der Estergruppe, beispielsweise mit Diisobutylaluminiumhydrid und anschließende Oxidation des erhaltenen primären Alkohols z.B. mit Braunstein oder Collinsreagenz führt zum Aldehyd der Formel Xₐ

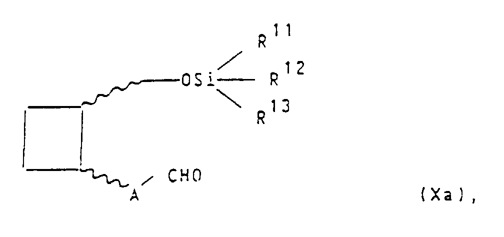

Die metallorganische Umsetzung des Aldehyds der Formel X mit einem Grignardreagenz der Formel XIa, worin B, D

X-Mg-B-D-R₄ (XIa),

und R⁴ die oben angegebenen Bedeutungen aufweisen und X Chlor, Brom oder Jod bedeutet. führt nach Schutz der Hydroxygruppe (beispielsweise durch Acylierung) und gegebenenfalls Diastereomerentrennung zu den Verbindungen der Formel XIIa

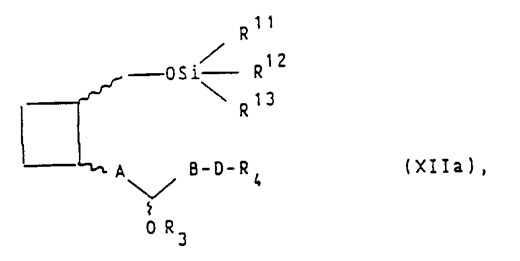

Die Herstellung der für die metallorganische Umsetzung benötigten Verbindung der Formel XIa erfolgt durch Reaktion des entsprechenden terminalen Halogenids mit Magnesium. Durch Umsetzung des Silylethers XIIa mit Tetrabutylammoniumfluorid und gegebenenfalls Diastereomerentrennung wird der Alkohol der Formel XIIIa erhalten.

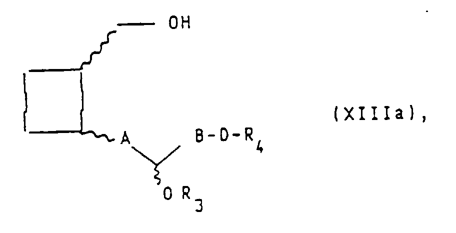

Die Oxidation der primären Alkoholgruppe in XIII z. B. Collinsreagenz oder Pyridiniumdichromat führt zum Aldehyd der Formel II.

Zu den Verbindungen der Formel XII, worin 8 eine CH₂-Gruppe und D eine CH=CR⁸-Gruppe bedeutet. kann man gelangen, beispielsweise durch eine metallorganische Umsetzung eines Propargylhalogenids und anschließende Alkylierung mit einem entsprechenden Alkylhalogenid und gegebenenfalls anschließende Lindlar-Hydrierung.

Ein alternativer Aufbau der unteren Kette geht von dem Aldehyd der Formel XIVa aus, der aus der Wittig-Horner-Umsetzung des Aldehyds VI und anschließender Reduktion und Oxidation resultierte.

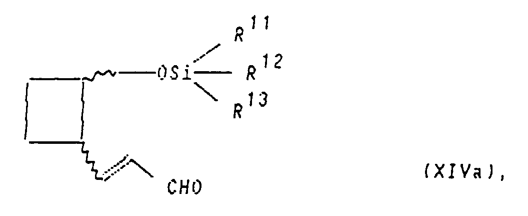

Wittig-Horner-Olefinierung des Aldehyds XIII mit einem Phosphonat der Formel XVa 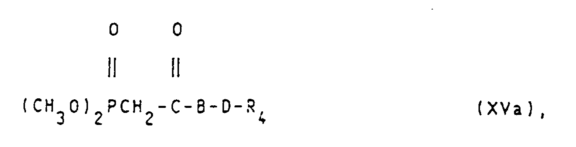 und Reduktion des entstandenen Ketons führte dann zum Alkohol der Formel XIIIa, der in die Diastereomeren getrennt werden kann.

Die Trennung der Enantiomeren und/oder Diastereomeren erfolgt nach den dem Fachmann bekannten Methoden, beispielsweise Hochdruckflüssigchromatographie an optisch aktiven Trägermaterialien.

Die als Ausgangsmaterial dienenden Verbindungen der Formel II mit m in der Bedeutung von 4 können beispielsweise hergestellt werden, indem man in an sich bekannter Weise Ester von cis- oder trans-Bis-1 ,2-dicarbonsäure-cycloheptan (bekannt durch zum Beispiel G. Sicher et al., Collection Czechoslov. Chem. Commun. 24, 262 (1961)) durch Reduktion in das Diol der Formel Vb überführt

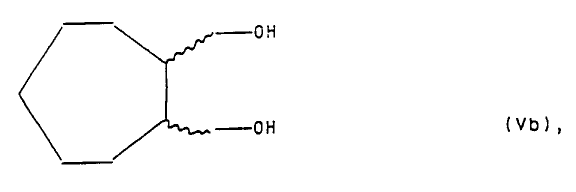

Das Diol der Formel Vb kann dann anschließend nach bekannten Methoden in den Monosilylether der Formel VIb 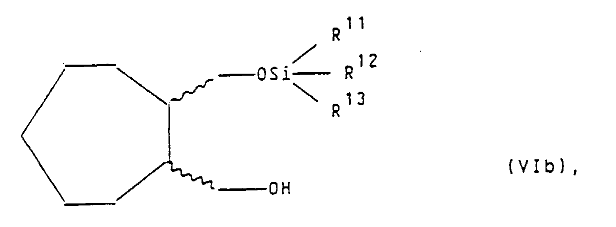 worin
R¹¹, R¹² und R¹³ gleich oder verschieden sind und C₁-C₄-Alkyl oder Phenyl bedeuten, überführt werden.

Durch Oxidation z.B. mit Collins-Reagenz oder durch das Swern-Verfahren (Tetrahedron 34, 1651 (1978)) wird der Aldehyd der Formel VIIb erhalten, 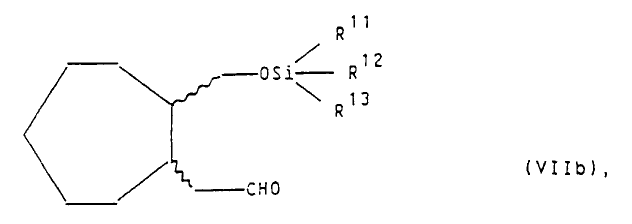 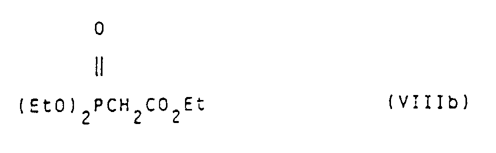 oder 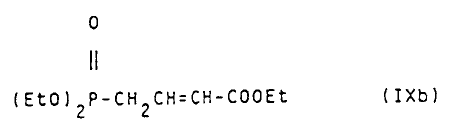 der in einer Wittig-Horner-Olefinierung mit dem Phosphonat der Formel VIII und einer Base und gegebenenfalls anschließender Hydrierung sowie anschließender Reduktion der Estergruppe, Oxidation des primären Alkohols, nochmaliger Wittig-Horner-Olefinierung mit dem Phosphonat der Formel VIII und gegebenenfalls anschließender Hydrierung in den Ester der Formel Xb 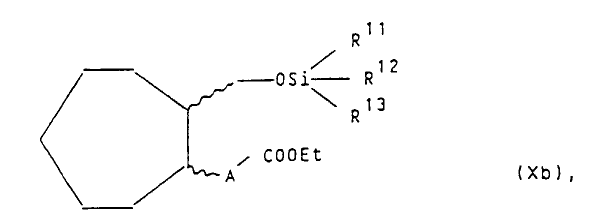 oder einer Wittig-Horner-Reaktion des Aldehyds der Formel VIIb mit einem Phosphonat der Formel IXb überführt wird (hierbei kann partiell eine cis/trans-Isomerisierung am Ringsystem auftreten, wobei die Isomeren auf späteren Stufen trennbar sind). wobei A die oben angegebene Bedeutung besitzt. Als Basen kommen beispielsweise Kaliumtert.-butylat, Diazabicycloundecan, Diazabicyclononan oder Natriumhydrid infrage. Reduktion der Estergruppe, beispielsweise mit Diisobutylaluminiumhydrid und anschließende Oxidation des erhaltenen primären Alkohols z.B. mit Braunstein oder Collinsreagenz führt zum Aldehyd der Formel XIb

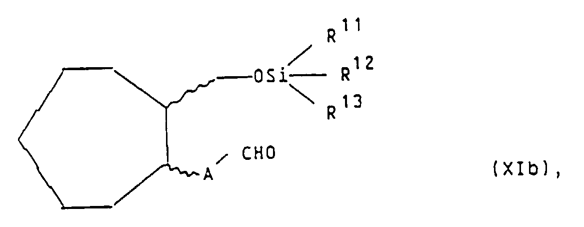

Die metallorganische Umsetzung des Aldehyds der Formel XI mit einem Grignardreagenz der Formel XIIb, worin B, D

X-Mg-B-D-R₄ (XIIb),

und R⁴ die oben angegebenen Bedeutungen aufweisen und X Chlor. Brom oder Jod bedeutet, führt nach Schutz der Hydroxygruppe und gegebenenfalls Diastereomerentrennung (beispielsweise durch Acylierung) zu den Verbindungen der Formel XIIIb

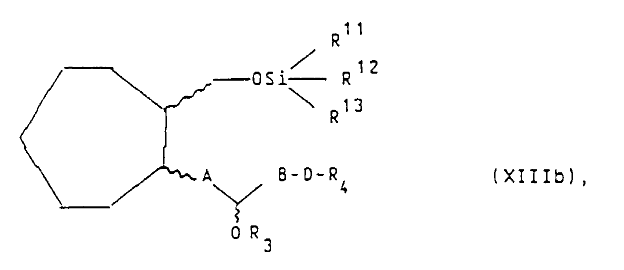

Die Herstellung der für die metallorganische Umsetzung benötigten Verbindung der Formel XIIb erfolgt durch Reaktion des entsprechenden terminalen Halogenids mit Magnesium. Durch Umsetzung des Silylethers XIIIb mit Tetrabutylammoniumfluorid und gegebenenfalls Diastereomerentrennung wird der Alkohol der Formel XIVb erhalten.

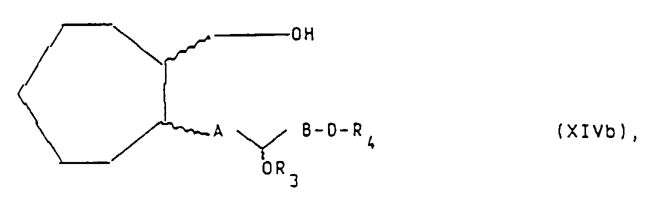

Die Oxidation der primären Alkoholgruppe in XIVb z. B. Collinsreagenz oder Pyridiniumdichromat führt zum Aldehyd der Formel II.

Zu den Verbindungen der Formel XIII, worin B eine CH₂-Gruppe und D eine CH=CR⁸-Gruppe bedeutet. kann man gelangen, beispielsweise durch eine metallorganische Umsetzung eines Propargylhalogenids und anschließende Alkylierung mit einem entsprecnenden Alkylhalogenid und gegebenenfalls anschließende Lindlar-Hydrierung.

Ein alternativer Aufbau der unteren Kette geht von dem Aldehyd der Formel XV aus, der aus der Wittig-Horner-Umsetzung des Aldehyds VIIb und anschließender Reduktion und Oxidation resultierte.

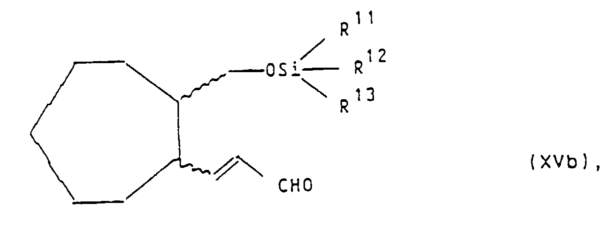

Wittig-Horner-Olefinierung des Aldehyds XV mit einem Phosphonat der Formel XVIb 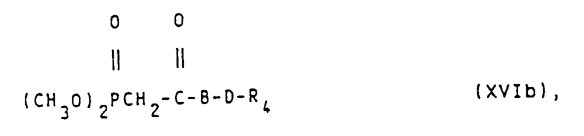 und Reduktion des entstandenen Ketons führte dann zum Alkohol der Formel XIIIb der in die Diastereomeren getrennt werden kann.

Der Einbau der chemisch und metabolisch labilen cis-Δ^{6,7}-Doppelbindung des LTB₄ in einen cis- oder trans-1,2-substituierten Cycloheptylring führt zu einer Stabilisierung, wobei insbesondere durch weitere Derivatisierung der funktionellen Gruppen, LTB₄-Derivate erhalten werden, die als LTB₄-Antagonisten wirken können.

Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise 0,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination. wie z.B mit Lipoxygenasehemmern. Cyclooxygenasehemmern, Prostacyclinagonisten, Thromboxanantagonisten, Leukotrien D₄-antagonisten, Leukotrien E₄-antagonisten, Leukotrien F₄-antagonisten, Phosphodiesterasehemmern, Calciumantagonisten oder PAF-Antagonisten verwendet werden.

Die nachfolgenden Ausführungsbeispiele dienen zur näheren Erläuterung des erfindungsgemäßen Verfahrens. In den Beispielen wurden nicht näher charakterisierte Diastereoisomere in der 5-Position als polar beziehungsweise unpolar charakterisiert (z. B. Diastereomer unpol (5)).

### BEISPIEL 1

### (+/-)-(5RS)-5-Acetoxy-5-[cis-(2RS)-2-((1E,3E)-(5RS)-5-acetoxy-1,3-tridecadienyl)-(1RS)-cyclobutyl]-pentansäure Diastereomer pol(5)

Zu 1,92 g Magnesium tropft man bei 25 °C unter Argon eine Lösung aus 8,9 g 4-Chlor-1-(tert.-butyldimethylsilyloxy)-butan in 8 ml Tetrahydrofuran, fügt einen Kristall Jod hinzu, erwärmt 10 Minuten auf 70 °C, rührt 30 Minuten bei 25 °C und verdünnt mit 25 ml Tetrahydrofuran.
Zu 30 ml dieser magnesiumorganischen Lösung tropft man bei -70 °C unter Argon eine Lösung aus 4,8 g cis-(1RS)-1-Formyl-(2RS)-2-[(1E,3E)-(5RS)-5-acetoxy-1,3-tridecadienyl]-cyclobutan in 40 ml Tetrahydrofuran und rührt 0,5 Stunden bei -70 °C. Man versetzt mit 50 ml gesättigter Ammoniumchloridlösung, extrahiert dreimal mit Diethylether, schüttelt die organische Phase mit halbkonzentrierter wässriger Natriumchlorid-Lösung, trocknet über wasserfreiem Natriumsulfat und dampft im Vakuum ein. Den Rückstand chromatographiert man an Kieselgel. Mit Hexan/Essigsäureethylester (8+2) erhält man 3 diastereomere 5-(tert.-Butyldimethylsilyloxy)-1-cis{2-[((1E,3E)-(5RS)-5-acetoxy-1,3-tridecadienyl]-cyclobutyl}-pentan-1-ol-Diastereomere in der Reihenfolge steigender Polaritäten.
0,7 g Diastereomer unpol(5), A
0,6 g Diastereomer unpol(5), B
3,6 g Diastereomer polar (5)
IR(CNCl₃): 3580, 2930, 2860, 1728, 1252, 995, 838 cm⁻¹

Zur Acetylierung fügt man zu einer Lösung aus 3,5 g des vorstehend beschriebenen, als (pol(5)-Diastereomer) bezeichneten Alkohols in 9 ml Pyridin 4,5 ml Essigsäureanhydrid und rührt 23 Stunden bei Raumtemperatur. Anschließend engt man im Vakuum unter Zusatz von Toluol ein und chromatographiert den Rückstand an Kieselgel. Mit Hexan/Diethylether (8+2) erhält man 3,7 g des entsprechenden Acetats als farbloses Öl. IR: 2930, 2862, 1728, 1610, 1375, 1254, 993, 839 cm⁻¹

Zur Silyletherspaltung rührt man 3,67 g des vorstehend hergestellten Acetats in 130 ml Tetrahydrofuran mit 3,67 g Tetrabutylammoniumfluorid 30 Minuten bei 0 °C und 3 Stunden bei 24 °C unter Argon. Anschließend verdünnt man mit Diethylether, wäscht dreimal mit Wasser, trocknet über wasserfreiem Magnasiumsulfat und dampft im Vakuum ein. Den Rückstand chromatographiert man mit Diethylether an Kieselgel. Dabei erhält man 2,84 mg des (+/-)-(5RS)-5-Acetoxy-5-[cis-(2RS)-2-((1E,3E)-(5RS)-5-acetoxy-1,3-tridecadienyl)-(1RS)-cyclobutyl]-pentan-1-ol als farbloses Öl.
IR: 3600 (breit), 2930, 2860, 1726, 1610, 1370, 1250, 990 cm⁻¹

Zur Oxidation der 1-Hydroxygruppe fügt man zu 2,76 g des vorstehend hergestellten Alkohols in 150 ml Dichlormethan bei 0 °C 16,4 g Collins-Reagenz (Bis-pyridin-chrom(VI)-oxid-Komplex; Tetrahedron Letters 1968, 3363).

und rührt 20 Minuten bei 0 °C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird ohne weitere Reinigung sofort weiter verarbeitet.

Zu einer Lösung von 2,47 g des so hergestellten Aldehyds in 50 ml Aceton tropft man unter Rühren bei -25 °C 5,3 ml Jones-Reagenz (Chrom(VI)-oxid in H₂SO₄).

(J.Chem.Soc. 1953, 2555) und rührt 15 Minuten bei -25 °C unter Argon. Anschließend fügt man 3 ml 2-Propanol zu, rührt 5 Minuten, verdünnt mit 250 ml Diethylether, schüttelt zweimal mit halbkonzentrierter wässriger Natriumchloridlösung, trocknet über wasserfreiem Magnesiumsulfat und dampft im Vakuum ein. Den Rückstand chromatographiert man an Kieselgel. Mit Hexan/Diethylether (1+1) erhält man 1,96 g der Titelverbindung als farbloses Öl.
IR: 3520(breit), 2930, 2860, 1728, 1375, 1250, 990, 948 cm⁻¹

Das Ausgangsmaterial für die obige Titelverbindung wird wie folgt hergestellt:

### 1a) 3-[cis-1-(tert.-Butyl-dimethylsilyloxymethyl)-cyclo-but-2-yl]-(2E)-propensäureethylester

Zu einer Lösung aus 2,8 g 2-Hydroxymethylcyclobutancarbonsäurelacton (C.C. Shroff et al., J.Org.Chem. 36 3356 (1971) in 70 ml Toluol tropft man bei 0 °C unter Argon 46 ml einer 1,2 molaren Lösung von Diisobutylaluminiumhydrid in Toluol und rührt 50 Minuten bei 0 °C. Anschließend tropft man 20 ml 2-Propanol und 23 ml Wasser zu, rührt 2 Stunden bei 22 °C, filtriert, wäscht mit Dichlormethan und dampft im Vakuum ein. Den Rückstand reinigt man durch Chromatographie an Kieselgel. Mit Essigsäureethylester/Hexan (4+1) erhält man 2,1 g cis-1,2-Dihydroxymethyl-cyclopentan als farblose Flüssigkeit.
IR: 3600, 3400, 2960, 1060 cm⁻¹

Zu einer Suspension von 2,93 g Natriumhydrid (als 55%ige Suspension in Mineralöl) in 130 ml Tetrahydrofuran tropft man bei 22 °C eine Lösung von 7,8 g des vorstehend hergestellten Diols in 12 ml Tetrahydrofuran und rührt 45 Minuten bei 22 °C. Man fügt anschließend 10 g tert.-Butyldimethylsilylchlorid zu, rührt 45 Minuten bei 22 °C und verdünnt dann mit ca. 0,8 Liter Diethylether. Man wäscht den Etherextrakt mit 10%iger wässriger Kaliumcarbonatlösung, schüttelt dreimal mit Wasser, trocknet mit wasserfreiem Natriumsulfat und dampft im Vakuum ein. Den Rückstand chromatographiert man an Kieselgel. Mit Hexan/Diethylether (95+5) erhält man 16,9 g cis-1-tert.-Butyldimethylsilyloxymethyl)-2-hydroymethyl-cyclobutan als farblose Flüssigkeit.
IR: 3420(breit); 2960, 2863, 1260, 840 cm⁻¹

Zu einer Lösung von 9 g des vorstehend beschriebenen Monosilylethers in 450 ml Dichlormethan fügt man bei 0 °C 70 g Collins-Reagenz (Chromsäure-Pyridin-Komplex) und rührt 30 Minuten bei 0 °C. Anschließend verdünnt man mit einer Mischung aus Hexan/Diethylether (3+2), fügt Celite hinzu, filtriert und dampft im Vakuum ein. Man erhält 8,7 g des Aldehyds, der ohne weitere Reinigung verwendet wird.
IR: 2958, 2930, 2860, 2740, 1713, 840 cm⁻¹

Zur Wittig-Olefinierung fügt man bei 24 °C 12 g Phosphonoessigsäuretriethylester und 7,2 g Diazabicycloundecen (DBU). Zu einer gerührten Suspension von 2,3 g Lithiumchlorid in 150 ml Acetonitril und rührt 15 Minuten. Anschließend tropft man eine Lösung von 8,7 g des vorstehend beschriebenen Aldehyds in 24 ml Acetonitrils zu, rührt 2,5 Stunden bei 24 °C und verdünnt dann mit Diethylether. Man schüttelt nacheinander mit Wasser, 10%iger wässriger Schwefelsäure und Wasser, trocknet mit wasserfreiem Natriumsulfat und dampft im Vakuum ein. Den Rückstand chromatographiert man mit Hexan/Diethylether (95+5) an Kieselgel. Dabei erhält man 9,0 g der Titelverbindung als farbloses Öl.
IR: 2950, 2860, 1710, 1650, 1260, 993, 840 cm⁻¹

### 1b) 5-[cis-1-(tert.-Butyl-dimethylsilyloxymethyl)-cyclo-but-2-yl]-(2E,4E)-pentadiensäure ethylester

Zu einer Lösung von 21 g des nach Beispiel 1a hergestellten α,β-ungesättigten Esters in 250 ml Toluol tropft man bei -70 °C unter Argon 129 ml einer 1,2 molaren Lösung von Diisobutylaluminiumhydrid in Toluol und rührt 30 Minuten bei -70 °C. Anschließend tropft man 30 ml 2-Propanol und dann 60 ml Wasser zu, rührt 2 Stunden bei 22 °C, filtriert, wäscht mit Dichlormethan und dampft im Vakuum ein. Man erhält 18,1 g 3-[cis-1-(tert.-Butyldimethylsilyloxy-methyl)-cyclo-but-2-yl]-(2E)-2-propen-1-ol der ohne weitere Reinigung verwendet wird.
IR: 3600, 3400, 2058, 840 cm⁻¹

Eine Lösung von 18 g des vorstehend hergestellten Alkohols in 400 ml Toluol versetzt man mit 61 g Braunstein und rührt 5 Stunden bei 24 °C. Anschließend wird filtriert, eingedampft und an Kieselgel chromatographiert. Mit Hexan/Diethylether (92+8) eluiert man 17,6 g des Aldehyds als farbloses Öl.
IR: 2950, 2860, 2740, 1680, 1633, 1470, 975, 840 cm⁻¹

Zur Wittig-Olefinierung fügt man bei 24 °C 19,4 g Phosphonoessigsäuretriethylester und 11,7 g Diazabicycloundecen zu einer gerührten Suspension von 3,66 g Lithiumchlorid in 255 ml Acetonitril und rührt 15 Minuten. Anschließend tropft man eine Lösung von 15,7 g des vorstehend beschriebenen α,β-ungesättigten Aldehyds in 40 ml Acetonitril zu, rührt 4 Stunden bei 24 °C und verdünnt dann mit Diethylether. Man schüttelt nacheinander mit Wasser, 10%iger wässriger Zitronensäure-Lösung und Wasser, trocknet mit wasserfreiem Natriumsulfat und dampft im Vakuum ein. Den Rückstand chromatographiert man mit Hexan/Diethylether (9+1) an Kieselgel. Dabei erhält man
11,9 g der Titelverbindung als farbloses Öl.
IR: 2958, 2860, 1703, 1638, 1615, 1255, 1003, 970, 837 cm⁻¹

### 1c) 5-[cis-1-(tert.-Butyl-dimethylsilyloxymethyl)-cyclo-but-2-yl]-(2E,4E)-pentadien-1-ol

Zu einer Lösung von 12 g des nach Beispiel 1b hergestellten Esters in 200 ml Toluol tropft man bei -70 °C unter Argon 76 ml einer 1,2 molaren Lösung von Diisobutylaluminiumhydrid in Toluol und 30 Minuten bei -70 °C. Anschließend tropft man 30 ml 2-Propanol und dann noch 40 ml Wasser zu, rührt 3 Stunden bei 23 °C, filtriert, wäscht mit Dichlormethan und dampft im Vakuum ein. Den Rückstand reinigt man durch Chromatographie an Kieselgel. Mit Hexan/Diethylether (8+2) erhält man 7,5 g des Alkohols als farbloses Öl.
IR: 3620, 3460, 838 cm⁻¹

Eine Lösung von 7,3 g des vorstehend hergestellten Alkohols in 150 ml Toluol versetzt man mit 23,3 g Braunstein und rührt 6 Stunden bei 24 °C. Anschließend wird filtriert, eingedampft und an Kieselgel chromatographiert. Mit Hexan/Diethylether (9+1) erhält man 5,4 g der Titelverbindung als farbloses Öl.
IR: 2955, 2858, 2740, 1683, 1634, 991, 842 cm⁻¹

### 1d) (5RS)-5-Acetoxy-1-[cis-1-(tert.-butyl-dimethylsilyl-oxymethyl)-cyclobut-2-yl]-(1E,3E)-tridecadien

Zu 1,12 g Magnesium in 4 ml Diethylether tropft man unter Erwärmen eine Lösung aus 8,9 g Octylbromid in 10 ml Diethylether und rührt 30 Minuten bei 25 °C.

Zu 9,6 ml (= 20,0 mMol) dieser Grignardlösung tropft man bei -20 °C unter Argon eine Lösung aus 5,1 g des nach Beispiel 1c hergestellten Aldehyds in 60 ml Diethylether und rührt 45 Minuten bei -20 °C. Man versetzt mit gesättigter wässriger Ammoniumchloridlösung, extrahiert dreimal mit Diethylether, schüttelt die organische Phase mit halbkonzentrierter wässriger Natriumchloridlösung, trocknet über wasserfreiem Natriumsulfat und dampft im Vakuum ein. Den Rückstand chromatographiert man an Kieselgel. Mit Hexan/Essigsäureethylester erhält man 7,0 g des entsprechenden Alkohols als Diastereomerengemisch.

Zur Acetylierung fügt man zu einer Lösung von 6,96 g des vorstehend hergestellten diastereomeren Alkoholgemisches in 20 ml Pyridin 10 ml Essigsäureanhydrid und rührt 24 Stunden bei Raumtemperatur. Anschließend engt man im Vakuum unter Zusatz von Toluol ein und chromatographiert den Rückstand an Kieselgel. Mit Hexan/Diethylether (95+5) erhält man 7,5 g der Titelverbindung (unpolares Diastereomeres) als farbloses Öl.
IR: 2938, 2860, 1725, 1655, 1250, 990, 838 cm⁻¹

### 1e) cis-(1RS)-1-Formyl-(2RS)-2-[(1E,3E)-(5RS)-5-acetoxy-1,3-tridecadienyl]-cydobutan

Zu einer Lösung von 7,5 g des nach Beispiel 1d hergestellten Acetats in 170 ml Tetrahydrofuran fügt man bei 0 °C 10,83 g Tetrabutylammoniumfluorid, rührt 15 Minuten bei 0 °C und 5,5 Stunden bei 24 °C. Anschließend verdünnt man mit 1Liter Diethylether und wäscht dreimal mit halbkonzentrierter wässriger Natriumchloridlösung. Man trocknet über wasserfreiem Magnesiumsulfat, dampft im Vakuum ein und chromatographiert den Rückstand an Kieselgel. Mit Hexan/Diethylether (7+3) eluiert man 5,2 g (5RS)-1-(cis-Hydroxymethyl-cyclobut-2-yl)-(1E,3E)-tridecadien als farbloses Öl.
IR: 3610, 3450, 2930, 2860, 1726, 1250, 992 cm⁻¹

Zu einer Lösung von 5,0 g des vorstehend hergestellten Alkohols in 140 ml Dichlormethan fügt man bei 0 °C 32 g Collins-Reagenz (Chromsäure-Pyridin-Komplex) und rührt 20 Minuten bei 0 °C. Anschließend verdünnt man mit einer Mischung aus Hexan/Diethylether (1+1), fügt Celite hinzu, filtriert und dampft im Vakuum ein. Der so erhaltene Aldehyd wurde ohne weitere Reinigung verwendet. (Rohausbeute 4,8 g)
IR: 2930, 2860, 2730, 1721, 1250, 990 cm⁻¹

### BEISPIEL 2

### (+/-)-(5RS)-5-Hydroxy-5-[cis-(2RS)-2-((1E,3E)-(5RS)-5-hydroxy-1,3-tridecadienyl)-(1RS)-cyclobutyl]-pentansäure Diastereomer pol(5)

Zu einer Lösung von 200 mg des nach Beispiel 1 hergstellten Diacetats (Diastereomer polar (5) ) in 4,5 ml Methanol fügt man 4,5 ml einer 0,5 molaren wäßrigen Lithiumhydroxidlösung und rührt 25 Stunden bei 50 °C. Anschließend säuert man mit einer 10%igen wässrigen Schwefelsäure auf pH 5 an, verdünnt mit Essigsäureethylester, schüttelt zweimal mit halbkonzentrierter wässriger Natriumchloridlösung, trocknet mit wasserfreiem Natriumsulfat und dampft im Vakuum ein. Den Rückstand chromatographiert man mit Diethylether/Ethanol (99+1) an Kieselgel. Dabei erhält man 148 mg der Titelverbindung als farbloses Öl.
IR: 3400, 2930, 2860, 1725, 1365, 1245, 992 cm⁻¹

### BEISPIEL 3

### (+/-)-(5RS)-5-Acetoxy-5-[cis-(2RS)-2-((1E,3E)-(5RS)-5-hydroxy-1,3-tridecadienyl)-(1RS)-cyclobutyl]-pentansäure Diastereomer pol(5)

Zu einer Lösung von 300 mg des nach Beispiel 1 hergestellten polaren diastereomeren Diacetats in 6,6 ml Methanol fügt man bei 23 °C 6,6 ml einer 0,5 normalen wässrigen Natronlauge und rührt 0,75 Stunden bei 23 °C unter Argon. Anschließend verdünnt man mit Wasser und säuert bei Eisbadtemperatur mit 10%iger wässriger Schwefelsäure auf pH 5 an. Man extrahiert mit Essigsäureethylester, wäscht zweimal mit halbkonzentrierter wässriger Natriumchloridlösung, trocknet mit wasserfreiem Natriumsulfat und dampft im Vakuum ein. Den Rückstand chromatographiert man an Kieselgel. Mit Diethylether/Hexan (8+2) erhält man 202 mg der Titelverbindung als farbloses Öl.
IR: 3600, 2925, 2858, 1729, 1712, 1700, 1250, 993, 960 cm⁻¹

### BEISPIEL 4

### (+/-)-(5RS)-5-Hydroxy-5-[cis-(2RS)-2-((1E,3E)-(5RS)-5-hydroxy-1,3-tridecadienyl)-(1RS)-cyclobutyl]-pentan-1-ol Diastereomer pol(5)

360 mg des in Beispiel 1 beschriebenen polaren diastereomeren Diacetats (Diastereomer pol(5) rührt man 60 Stunden bei 24 °C mit 11 ml einer Lösung von Kaliumhydroxid in Wasser und Ethanol (Herstellung: man löst 5 g Kaliumhydroxid in 67,5 ml Wasser und 182,5 ml Ethanol). Anschließend säuert man mit 10%iger wässriger Zitronensäurelösung auf pH 6 an, extrahiert viermal mit je 20 ml Dichlormethan, schüttelt die organische Phase mit Halbkonzentrierter wässriger Natriumchloridlösung, trocknet über wasserfreiem Magnesiumsulfat und dampft im Vakuum ein. Den Rückstand chromatographiert man mit Essigsäureethylester an Kieselgel. Dabei erhält man 103 mg der Titelverbindung als farbloses Öl.
IR: 3605, 3360 (breit), 2930, 2860, 993 cm⁻¹

### BEISPIEL 5

### (+/-)-(5RS)-5-Acetoxy-5-[cis-(2RS)-2-((1E,3E)-(5RS)-5-acetoxy-1,3-tridecadienyl)-(1RS)-cyclobutyl]-pentansäure Diastereomer unpol(5), A

Zur Acetylierung fügt man zu einer Lösung aus 0,61 g des in Beispiel 1 beschriebenen unpolaren Alkohols (Diastereomer unpol(5), A) in 2,0 ml Pyridin 1 g Essigsäureanhydrid und rührt 22 Stunden bei Raumtemperatur. Anschließend engt man im Vakuum unter Zusatz von Toluol ein und chromatographiert den Rückstand an Kieselgel. Mit Hexan/Diethylether (9+1) erhält man 0,62 g des Acetats als farbloses Öl.
IR: 2930, 2860, 1726, 1606, 1374, 1252, 993, 840 cm⁻¹

Zur Silyletherspaltung rührt man 0,61 g des vorstehend hergestellten Acetats in 25 ml Tetrahydrofuran mit 0,7 g Tetrabutylammoniumfluorid 20 Minuten bei 0 °C und 4 Stunden bei 24 °C unter Argon. Anschließend verdünnt man mit Diethylether, wäscht dreimal mit Wasser, trocknet über wasserfreiem Magnesiumsulfat und dampft im Vakuum ein. Den Rückstand chromatographiert man mit Diethylether/Hexan (8+2) an Kieselgel. Dabei erhält man 1,3 g des 1-Alkohols als farbloses Öl.
IR: 3620, 3450, 2930, 2860, 1725, 1608, 1375, 1250, 990 cm⁻¹

Zur Oxidation der 1-Hydroxygruppe fügt man zu 0,43 g des vorstehend hergestellten Alkohols in 50 ml Dichlormethan 2,5 g Collins-Reagenz und rührt 20 Minuten bei 0 °C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird sofort ohne weitere Reinigung weiter verarbeitet.

Zu einer Lösung von 0,41 g des vorstehend hergestellten Aldehyds in 10 ml Aceton tropft man unter Rühren bei -25 °C 0,9 ml Jones-Reagenz (J.Chem.Soc. 1953, 2555) und rührt 15 Minuten bei -25 °C unter Argon. Anschließend fügt man 2 ml 2-Propanol zu, rührt 5 Minuten, verdünnt mit 60 ml Diethylether, schüttelt zweimal mit halbkonzentrierter wässriger Natriumchloridlösung, trocknet über wasserfreiem Natriumsulfat und dampft im Vakuum ein. Den Rückstand chromatographiert man an Kieselgel. Mit Diethylether/Hexan (7+3) erhält man 374 mg der Titelverbindung als farbloses Öl.
IR: 3520 (breit), 2930, 2859, 1725, 1372, 1250, 990 cm⁻¹

Das Ausgangsmaterial für die obige Titelverbindung wird wie folgt hergestellt:

### BEISPIEL 6

### (+/-)-(5RS)-5-Hydroxy-5-[cis-(2RS)-2-((1E,3E)-(5RS)-5-hydroxy-1,3-tridecardi enyl)-(1RS)-cyclobutyl]-pentansäure Diastereomer unpol(5) A

In Analogie zu Beispiel 2 erhält man aus 180 mg des nach Beispiel 5 hergestellten Diacetats 124 mg der Titelverbindung als farbloses Öl.
IR: 3400, 2930, 2858, 1725, 1360, 1250, 992, 930 cm⁻¹

### BEISPIEL 7

### (+/-)-(5RS)-5-Acetoxy-5-[cis-(2RS)-2-((1E,3E)-(5RS)-5-hydroxy-1,3-tridecadienyl)-(1RS)-cyclobutyl]-pentansäure Diastereomer unpol(5) A

In Analogie zu Beispiel 3 erhält man aus 150 mg des nach Beispiel 5 hergestellten Diacetats 123 mg der Titelverbindung als farbloses Öl.
IR: 3600, 2930, 2860, 1721, 1252, 992, 962 cm⁻¹

### BEISPIEL 8

### (+/-)-(5RS)-5-Hydroxy-5-[cis-(2RS)-2-((1E,3E)-(5RS)-5-hydroxy-1,3-tridecadienyl)-(1RS)-cyclobutyl]-pentan-1-ol Diastereomer unpol(5)A

In Analogie zu Beispiel 4 erhält man aus 170 mg des nach Beispiel 5 hergestellten Diacetats 95 g der Titelverbindung als farbloses Öl.
IR: 3600, 3380 (breit), 2930, 2860, 992 cm⁻¹

### BEISPIEL 9

### (+/-)-(5RS)-5-Acetoxy-5-[trans-(2RS)-2-((1E,3E)-(5RS)-5-acetoxy-1,3-tridecadienyl)-(1RS)-cyclobutyl]-pentansäure Diastereomer B

Zu 1,92 g Magnesium tropft man bei 25 °C unter Argon eine Lösung aus 8,9 g 4-Chlor-1-(tert.-butyldimethylsilyloxy)-butan in 8 ml Tetrahydrofuran, fügt einen Kristall Jod hinzu, erwärmt 10 Minuten auf 60 °C, rührt 30 Minuten bei 25 °C und verdünnt mit 25 ml Tetrahydrofuran.

Zu 25 ml dieser magnesiumorganischen Lösung tropft man bei -70 °C unter Argon eine Lösung aus 4,0 g trans-(1RS)-1-Formyl-(2RS)-2-[(1E,3E)-(5RS)-5-acetoxy-1,3-tridecadienyl-cyclobutan in 40 ml Tetrahydrofuran und rührt 0,5 Stunden bei -70 °C. Man versetzt mit 100 ml gesättigter wässriger Ammoniumchloridlösung, extrahiert mit Diethylether, schüttelt die organische Phase mit halbkonzentrierter wässriger Natriumchloridlösung, trocknet über wasserfreiem Natriumsulfat und dampft im Vakuum ein. Den Rückstand chromatographiert man an Kieselgel. Mit Hexan/Essigsäureethylester (85+15) erhält man 2 5-(tert-Butyldimethylsilyloxy-1-trans-{2-[1E,3E-(5RS)-5-acetoxy-1,3-tridecandienyl]-cyclopentyl}-pentan-1-ol-Diastereomere in der Reihenfolge steigender Polarität.
1,94 g unpolares Diastereomeres A
1,7 g polares Diastereomeres B
IR: 3430, 2932, 2859, 1725, 1372, 1256, 992, 839 cm⁻¹

Zur Acetylierung fügt man zu einer Lösung aus 0,9 g des vorstehend hergestellten Alkohols (Diastereomer B) in 20 ml Pyridin 1,0 ml Essigsäureanhydrid und rührt 21 Stunden bei Raumtemperatur. Anschließend engt man im Vakuum unter Zusatz von Toluol ein und chromatographiert den Rückstand an Kieselgel. Mit
Hexan/Essigsäureethylester (85+15) erhält man 0,97 g des Acetats als farbloses Öl.
IR: 2940, 2862, 1728, 1375, 1257, 992, 840 cm⁻¹

Zur Silyletherspaltung rührt man 0,93 g des vorstehend hergestellten Acetats in 36 ml Tetrahydrofuran mit 1,1 g Tetrabutylammoniumfluorid 1 Stunde bei 0 °C und 3 Stunden bei 24 °C unter Argon. Anschließend verdünnt man mit Diethylether, wäscht dreimal mit Wasser, trocknet über wasserfreiem Magnesiumsulfat und dampft im Vakuum ein. Den Rückstand chromatographiert man an Kieselgel. Mit Hexan/Essigsäureethylester erhält man 0,65 g des (+/-)-(5RS)-5-Acetoxy-5-[trans-(2RS)-2-((1E,3E)-(5RS)-5-acetoxy-1,3-tridecadienyl)-(1RS)-cyclobutyl]-pentan-1-ol als farbloses Öl.
IR: 3620, 3460, 2730, 2860, 1725, 1608, 1375, 1250, 992, 950 cm⁻¹

Zur Oxidation der 1-Hydroxygruppe fügt man zu 0,64 g des vorstehend hergestellten Alkohols (Diastereomer B) in 70 ml Dichlormethan bei 0 °C 3,8 g Collins-Reagenz und rührt 20 Minuten bei 0 °C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird sofort ohne weitere Reinigung verwendet.

Zu einer Lösung von 0,6 g des vorstehend hergestellten Aldehyds in 16 ml Aceton tropft man unter Rühren bei -25 °C 1,3 ml Jones-Reagenz und rührt 15 Minuten bei -25 °C unter Argon. Anschließend fügt man 3 ml 2-Propanol zu, rührt 5 Minuten, verdünnt mit 100 ml Diethylether schüttelt zweimal mit halbkonzentrierter wässriger Natriumchloridlösung, trocknet über wasserfreiem Natriumsulfat und dampft im Vakuum ein. Den Rückstand chromatographiert man an Kieselgel. Mit Hexan/Essigsäureethylester (6+4) erhält man 0,52 g der Titelverbindung als farbloses Öl.
IR: 3520, 2930, 2860, 1725, 1658, 1360, 1250, 990, 946 cm⁻¹

### 9a) 5-[trans-1-(tert.-Butyl-dimethylsilyloxymethyl)-cyclobut-2-yl]-(2E,4E)-pentadiensäure ethylester

Eine Lösung von 20 g trans-1,2-Cyclobutandicarbonsäuredichlorid in 40 ml Tetrahydrofuran tropft man vorsichtig bei -15 °C zu einer Lösung von 8,4 g Natriumborhydrid in 280 ml Ethylalkohol. Nach Beendigung der Gasentwicklung rührt man 30 Minuten bei -15 °C nach und tropft dann langsam 150 ml Wasser dazu. Man rührt 30 Minuten bei 22 °C und verdünnt dann mit 1,5 Liter Diethylether. Man schüttelt zweimal mit Wasser, zweimal mit halbkonzentrierter wässriger Natriumchloridlösung, trocknet über wasserfreiem Natriumsulfat und dampft im Vakuum ein. Den Rückstand reinigt man durch Chromatographie an Kieselgel. Mit Essigsäureethylester erhält man 9,5 g trans-1,2-Dihydroxymethyl-cyclopentan als farblose Flüssigkeit.
IR: 3610, 3400, 2960, 1062 cm⁻¹

Zu einer Lösung von 19,3 g trans-1,2-Dihydroxymethyl-cyclopentan in 200 ml Dimethylformamid fügt man bei 0 °C 22,7 g Imidazol und 25,1 g tert.-Butyldimethylsilylchlorid und rührt 22 Stunden bei 24 °C. Man verdünnt mit 1,5 Liter Diethylether, schüttelt zweimal mit je 80 ml 10%ige wässrige Schwefelsäure, wäscht mit Wasser neutral, trocknet über wasserfreiem Magnesiumsulfat und dampft im Vakuum ein. Den Rückstand chromatographiert man an Kieselgel. Mit Hexan/Essigsäureethylester (8+2) erhält man 17 g trans-1-(tert.-Butyl-dimethylsilyloxymethyl)-2-hydroxymethylcyclopentan als farblose Flüssigkeit.
IR: 3420, 2960, 2860, 1260, 840 cm⁻¹
Zu einer Lösung von 14,9 g des vorstehend beschriebenen Monosilylethers in 750 ml Dichlormethan fügt man 70 g Collins-Reagenz und rührt 30 Minuten bei 0 °C. Anschließend verdünnt man mit einer Mischung aus Hexan/Diethylether (3+2), fügt Celite hinzu, filtriert und dampft im Vakuum ein. Man erhält 13,8 g des Aldehyds, der ohne weitere Reinigung verwendet wird.
IR: 2958, 2860, 2720, 1719, 840 cm⁻¹

Zur Wittig-Homer-Olefinierung fügt man bei 24 °C 8 g Phosphonocrotonsäuretriethylester und 4,9 g Diazabicycloundecen (DBU) zu einer gerührten Suspension von 1,37 g Lithiumchlorid in 250 ml Acetonitril und rührt 10 Minuten. Anschließend tropft man eine Lösung aus 6,5 g des vorstehend beschriebenen Aldehyds in 50 ml Acetonitril hinzu, rührt 3,5 Stunden bei 24-°C und verdünnt dann mit Diethylether. Man schüttelt nacheinander mit Wasser, 10%iger wässriger Zitronensäure-Lösung und Wasser, trocknet über wasserfreiem Magnesiumsulfat und dampft im Vakuum ein. Den Rückstand chromatographiert man mit Hexan/Essigsäureethylester an Kieselgel. Dabei erhält man 5,9 g der Titelverbindung als farbloses Öl.
IR: 2958, 2860, 1702, 1639, 1620, 1470, 1255, 1003, 840 cm⁻¹

### 9b) 5-[trans-1-tert.-Butyl-dimethylsilyloxymethyl)-cydobut-2-yl]-(2E,4E)-pentadien-1-ol

Zu einer Lösung von 5,2 g des nach Beispiel 9a) hergestellten Esters in 150 ml Toluol tropft man bei -70 °C unter Argon 33,5 ml einer 1,2 molaren Lösung von Diisobutylaluminiumhydrid in Toluol und rührt 30 Minuten bei -70 °C. Anschließend tropft man 16 ml 2-Propanol und nach 5 Minuten 16 ml Wasser zu, rührt 2,5 Stunden bei 23 °C, filtriert, wäscht mit Dichlormethan und dampft im Vakuum ein. Den Rückstand reinigt man durch Chromatographie an Kieselgel. Mit Hexan/Diethylether (7+3) erhält man 4,85 g des Alkohols als farbloses Öl.
IR: 3610, 3450, 990, 840 cm⁻¹

Zur Aldehydherstellung versetzt man eine Lösung von 4,8 g des vorstehend hergestellten Alkohols in 100 ml Toluol mit 15 g Braunstein und rührt 3 Stunden bei 24 °C. Anschließend wird filtriert, eingedampft und an Kieselgel chromatographiert. Mit Hexan/Essigsäureethylester (8+2) erhält man 4,5 g der Titelverbindung als farbloses Öl.
IR: 2960, 2860, 2740, 1680, 1633, 990, 940, 840 cm⁻¹

### 9c) (5RS)-5-Acetoxy-1-[trans-1-(tert.-butyl-dimethylsilyl-oxymethyl)-cyclobut-2-yl]-(lE,3E)-tridecadien

Zu 1,12 g Magnesium in 4 ml Diethylether tropft man unter Erwärmen eine Lösung aus 8,9 g Octylbromid in 10 ml Diethylether und rührt 30 Minuten bei 25 °C.

Zu 8,33 ml (= mMol) dieser Grignardlösung tropft man bei -20 °C unter Argon eine Lösung aus 4,4 g des nach Beispiel 9 b hergestellten Aldehyds in 50 ml Diethylether und rührt 45 Minuten bei -20 °C. Man versetzt mit gesättigter Ammoniumchloridlösung, extrahiert dreimal mit Diethylether, schüttelt die organische Phase mit halbkonzentrierter wässriger Natriumchloridlösung, trocknet über wasserfreiem Natriumsulfat und dampft im Vakuum ein. Den Rückstand chromatographiert man an Kieselgel. Mit Hexan/Essigsäureethylester (6+4) erhält man 6,1 g des Alkohols (Diastereomerengemisch) als farbloses Öl.

Zur Acetylierung fügt man zu einer Lösung von 6,05 g des vorstehend hergestellten Alkohols in 20 ml Pyridin 10 ml Essigsäureanhydrid und rührt 23 Stunden bei Raumtemperatur. Anschließend engt man im Vakuum unter Zusatz von Toluol ein und chromatographiert den Rückstand an Kieslgel. Mit Hexan/Essigsäureethylester (9+1) erhält man 6,4 g der Titelverbindung als farbloses Öl.
IR: 2938, 2860, 1725, 1658, 1255, 991, 945, 840 cm⁻¹

### 9d) trans-(1RS)-1-formyl-(2RS)-2-[(1E,3E)-(5RS)-5-acetoxy-1,3-tridecadienyl]-cyclobutan

Zu einer Lösung von 6,3 g des nach Beispiel 9c hergestellten Acetats in 160 ml Tetrahydrofuran fügt man bei 0 °C 9,2 g Tetrabutylammoniumfluorid, rührt 15 Minuten bei 0 °C und 3-Stunden bei 24 °C. Anschließend verdünnt man mit 1 l Diethylether und wäscht dreimal mit halbkonzentrierter wässriger Natriumchloridlösung. Man trocknet mit wasserfreiem Natriumsulfat, dampft im Vakuum ein und chromatographiert den Rückstand an Kieselgel. Mit Hexan/Essigsäureethylester (7+3) eluiert man 4,4 g des Alkohols als farbloses Öl.
IR: 3625, 3450, 2932, 2862, 1728, 1680, 1250, 991 cm⁻¹

Zu einer Lösung von 4,3 g des vorstehend hergestellten Alkohols in 130 ml Dichlormethan fügt man bei 0 °C 28 g Collins-Reagenz und rührt 20 Minuten bei 0 °C. Anchließend verdünnt man mit einer Mischung aus Hexan/Diethylether (1+1), fügt Celite hinzu, filtriert und dampft im Vakuum ein. Der so erhaltene Aldehyd wurde ohne weitere Reinigung verwendet.
IR: 2930, 2860, 2721, 1721, 1250, 990 cm⁻¹

### BEISPIEL 10

### (+/+)-(5RS)-5-Hydroxy-5-[trans-(2RS)-2-((1E,3E)-(5RS)-5-hydroxy-1,3-tridecadienyl)-(1RS)-cyclobutyl]-pentansäure Diastereomer B

In Analogie zu Beispiel 2 erhält man aus 180 mg des nach Besipiel 9 hergestellten Diacetats 134 mg der Titelverbindung als farbloses Öl.
IR (Film): 3450, 2928, 2858, 1714, 1660, 1250, 989, 930 cm⁻¹

### BEISPIEL 11

### (+/-(5RS)-5-Acetoxy-5-[trans-(2RS)-2-((1E,3E)-(5RS)-5-hydroxy-1,3-tridecadienyl)-(1RS)-cyclobutyl]-pentansäure Diastereomer B

In Analogie zu Beispiel 3 erhält man aus 200 mg des nach Beispiel 9 hergestellten Diacetats 46 mg der Titelverbindung als farbloses Öl.
IR (Film): 3450, 2930, 2860, 1730, 1710, 1660, 1275, 990 cm⁻¹

### BEISPIEL 12

### (+/-)-(5RS)-5-Acetoxy-5-[trans-(2RS)-2-((1E,3E)-(5RS)-5-acetoxy-1,3-tridecadienyl)-(1RS)-cyclobutyl]-pentansäure Diastereomer A

Zu einer Lösung von 0,64 g des nach Beispiel 9 aus dem unpolaren Alkohol (Diastereomer-A) durch Acetylierung und Silyletherspaltung hergestellten 1-Alkohols in 70-ml Dichlormethan fügt man bei 0 °C 3,8 g Collinsreagenz und rührt 20 Minuten bei 0 °C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird sofort ohne weitere Reinigung verwendet.

Zu einer Lösung von 0,6 g des vorstehend hergestellten Aldehyds in 16 ml Aceton tropft man unter Rühren bei -25 °C 1,3 ml Jones-Reagenz und rührt 12 Minuten bei -25 °C unter Argon. Anschließend fügt man 3 ml 2-Propanol zu, rührt 5 Minuten, verdünnt mit Diethylether, schüttelt mit halbkonzentrierter wässriger Natriumchloridlösung, trocknet über wasserfreiem Natriumsulfat und dampft im Vakuum ein. Den Rückstand chromatographiert man an Kieselgel. Mit Hexan/Essigsäureethylester (6+4) erhält man 0,52 g der Titelverbindung als farbloses Öl.
IR : 3520, 2930, 2860, 1724, 1658, 1373, 1250, 989, 946 cm⁻¹

### BEISPIEL 13

### (+/-)-(5RS)-5-Hydroxy-5-[trans-(2RS)-2-((1E,3E)-(5RS)-5-hydroxy-1,3-tridecadienyl)-(1RS)-cyclobuyl]-pentansäure Diastereomer A

In Analogie zu Beispiel 2 erhält man aus 200 mg des nach Beispiel 12 hergestellten Diacetats 153 mg der Titelverbindung als farbloses Öl.
IR : 3600, 3420, 2930, 2859, 1730, 1660, 1250, 990 cm⁻¹

### BEISPIEL 14

### (+/-)-(5RS)-5-Acetoxy-5-[trans-(2RS)-2-((1E,3E)-(5RS)-5-hydroxy-1,3-tridecadienyl)-(1RS)-cyclobutyl]-pentansäure Distereomer A

In Analogie zu eispiel 3 erhält man aus 200 mg des nach eispiel 12 hergestellten Diacetats 174-mg der Titelverbindung als farbloses Öl.
IR : 3610, 3450, 2930, 2860, 1724, 1660, 1250, 990 cm⁻¹

### BEISPIEL 15

### (+/-)-(5RS)-5-Hydroxy-5-[cis-(2RS)-2-((1E,3E)-(5RS)-5-hydroxy-1,3-tridecadienyl)-(1RS)-cyclobutyl]-pentansäuremethylester Distereomer pol(5)

Zu einer Lösung von 100 mg der nach Beispiel 2 hergestellten Säure in 10 ml Dichlormethan tropft man bei 0 °C eine etherische Diazomethanlösung bis zur bleibenden Gelbfärbung und rührt 15 Minuten bei 0 °C. Anschließend dampft man im Vakuum ein und chromatographiert den Rückstand an Kieselgel. mit Hexan/Essigsäureethylester (2+8) erhält man 69 mg der Titelverbindung als farbloses Öl.
IR (Film): 3600, 2925, 2855, 1738, 1658, 990 cm⁻¹

### BEISPIEL 16

### (+/-)-(5RS)-5-Hydroxy-5-[cis-(2RS)-2-((1E,3E)-(5RS)-5-hydroxy-1,3-tridecadienyl)-(1RS)-cyclobutyl]-pentansäuremethylester Distereomer unpol(5) A

In Analogie zu Beispiel 15 erhält man aus 110 mg der nach Beispiel 6 hergestellten Säure 95 mg der Titelverbindung als Öl.
IR (Film): 3610, 2925, 2855, 1738, 1660, 990 cm⁻¹

### BEISPIEL 17

### (+/-)-(5RS)-5-Acetoxy-5-[cis-(2RS)-2-((1E,3E)-(5RS)-5-hydroxy-1,3-tridecadienyl)-(1RS)-cyclobutyl]-pentansäuremethylester Diastereomer pol(5)

In Analogie zu Beispiel 15 erhält man aus 70 mg der nach Beispiel 3 hergestellten Säure 71 mg der Titelverbindung als farbloses Öl.
IR (Film): 3400, 2924, 2858, 1739, 1658, 1240, 990 cm⁻¹

### BEISPIEL 18

### (+/-)-(5RS)-5-Hydroxy-5-[cis-(2RS)-2-((1E,3E)-(5RS)-5-hydroxy-1,3-tridecadienyl)-(1RS)-cyclobutyl]-pentansäure-tris-(hydroxymethyl)-aminomethansalz Diastereomer pol(5)

Zu einer Lösung von 40 mg der nach Beispiel 2 hergestellten Carbonsäure in 6 ml Acetonitril fügt man bei 70 °C eine Lösung von 15 mg Tris-(hydroxymethyl)aminomethan in 0,03 ml Wasser. Man läßt unter Rühren abkühlen, decantiert nach 16 Stunden vom Lösungsmittel ab und trocknet den Rückstand im Vakuum. Man isoliert 16 mg der Titelverbindung als wachsartige Masse.

### BEISPIEL 19

### (+/-)-(5RS)-5-Hydroxy-5-[cis-(2RS)-2-((1E,3E)-(5RS)-5-hydroxy-1,3-tridecadienyl)-(1RS)-cyclobutyl]-pentansäure-1,5-lacton Distereomer pol(5)

Zu einer Lösung von 53 mg der nach Beispiel 6 hergestellten Carbonsäure in 9 ml Toluol fügt man bei 24 °C über einen Zeitraum von 24 Stunden portionsweise 0,9 g wasserfreies Magnesiumsulfat und rührt weitere 24 Stunden bei 24 °C. Anschließend filtriert man und chromatographiert den Eindampfrückstand an Kieselgel. Mit Toluol/Essigsäureethylester (7+3) eluiert man 28 mg des 1,5-Lactons als farbloses Öl.
IR : 3600, 2930, 2860, 1729, 1250, 990 cm⁻¹

### Beispiel 20

### (+/-)-(5RS)-5-Acetoxy-5-[cis-(2RS)-2-((1E,3E)-(5R oder 5S)-5-acetoxy-1,3-tridecadienyl)-(1RS)-cycloheptyl]-pentansäure (Diastereomer A)

Zu 238 mg Magnesium tropft man bei 24 °C unter Argon eine Lösung aus 1.09 g 4-Chlor-1-(tert.-butyl-dimethylsilyloxy)-butan in 1 ml Tetrahydrofuran, fügt einen Kristall Iod hinzu, erwärmt 10 Minuten auf 70 °C, rührt 30 Minuten bei 24 °C und verdünnt mit 3 ml Tetrahydrofuran.

Zu einer Lösung von 467 mg cis-(1RS)-1-Formyl-(2RS)-2-[(1E,3E)-(5R oder 5S)-5-acetoxy-1,3-tridecadienyl-cycloheptan (Diastereomer A) aus Beispiel 1e) in 2.6 ml Tetrahydrofuran tropft man bei -70 °C unter Argon die obig hergestellte Grignard-Lösung, läßt auf -30 °C erwärmen und rührt 1 Stunde bei dieser Temperatur. Man gibt die Reaktionsmischung auf 100 ml gesättigte wässrige Ammoniumchlorid-Lösung und extrahiert dreimal mit je 100 ml Essigsäureethylester. Die vereinigten organischen Phasen werden einmal mit halbkonzentrierter wässriger Natriumchlorid-Lösung gewaschen, über wasserfreiem Natriumsulfat getrocknet und nach Filtration im Vakuum eingeengt. Den so erhaltenen Rückstand reinigt man durch Chromatographie an Kieselgel. Mit Hexan/Methyl-tert.-Butylether (92 + 8) erhält man 351 mg 5-(tert.-Butyldimethylsilyloxy)-1-cis-{2-[(1E,3E)-(5RS)-5-acetoxy-1,3-tridecadienyl]-cycloheptyl}-pentan-1-ol als farbloses Öl.
IR (CHCl₃): 3690, 3610, 3440 (breit), 3005, 2932, 2860, 1727, 1465, 1375, 1247, 1098, 995, 840 cm⁻¹.

Zur Acetylierung gibt man zu einer Lösung aus 345 mg des vorstehend beschriebenen Alkohols in 1 ml Pyridin 0.5 ml Acetanhydrid und rührt 6 Stunden bei 24 °C. Anschließend verdünnt man mit 1 ml Wasser und engt im Vakuum unter Toluolzusatz ein. Den so erhaltenen Rückstand reinigt man durch Chromatographie an Kieselgel. Mit Hexan / 0-10% Essigsäureethylester erhält man 347 mg des Acetats als farbloses Öl.
IR (CHCl₃): 3002, 2930, 2860, 1725, 1464, 1374, 1243, 1100, 992, 838 cm⁻¹.

Zur Silyletherspaltung rührt man 344 mg des vorstehend hergestellten Acetats in 2.3 ml Tetrahydrofuran mit 366 mg Tetrabutylammoniumfluoridtrihydrat bei 24 °C unter Argon für 3 Stunden. Anschließend verdünnt man mit 100 ml Diethylether, wäscht dreimal mit je 20-ml Wasser, einmal mit gesättigter wässriger Natriumchlorid-Lösung, trocknet über wasserfreiem Natriumsulfat und engt nach Filtration im Vakuum ein. Man erhält 314 mg des (+/-)-(5RS)-5-Acetoxy-5-[cis-(2RS)-2-((1E,3E)-(5RS)-5-acetoxy-1,3-tridecadienyl-(1RS)-cycloheptyl]-pentan-1-ol als Öl. Dieser wird ohne weitere Reinigung in 15.5 ml Dichlormethan gelöst, unter Argon bei 0 °C mit 2.63 g Collins-Reagenz (Bis-pyridin-Chrom(VI)-oxid-Komplex, Tetrahedron Letters 1968, 3363) versetzt und 1 Stunde bei 0 °C gerührt. Anschließend wird mit Diethylether verdünnt, mit Celite versetzt, über Celite abfiltriert und mit Diethylether gut nachgewaschen. Der nach dem Einengen erhaltene 1-Aldehyd wird ohne weitere Reinigung sofort in 13.8 ml Aceton gelöst, bei -30 °C unter Argon mit 0.35 ml Jones-Reagenz (Chrom(VI)oxid in H₂SO₄) (J. Chem. Soc. 1953, 2555) versetzt und 20 Minuten bei dieser Temperatur gerührt. Anschließend gibt man 0.15-ml 2-Propanol hinzu, rührt 10 Minuten, verdünnt mit 70 ml Essigsäureethylester, schüttelt viermal mit je 20 ml halbkonzentrierter wässriger Natriumchlorid-Lösung, trocknet über wasserfreiem Natriumsulfat und dampft nach Filtration im Vakuum ein. Den so erhaltenen Rückstand reinigt man durch Chromatographie an Kieselgel. Mit Hexan / 0-50% Essigsäureethylester erhält man 117 mg der Titelverbindung als farbloses Öl.
IR (Film): 3450, 3190 (breit), 3022, 2925, 2855, 1737, 1710, 1456, 1370, 1240, 990 cm⁻¹.

Das Ausgangsmaterial für die obige Titelverbindung wird wie folgt hergestellt:

### 20a) cis-1,2-Bis-(hydroxymethyl)-cycloheptan

13.0 g cis-1,2-Cycloheptandicarbonsäureanhydrid (J. Sicher et al., Collection Czechoslov. Chem. Commun. 26, 262 (1961)) werden in 13.8 ml Toluol und 18.8 ml Methanol gelöst. Nach Zugabe von 128 mg p-Toluolsulfonsäure wird 20 Stunden unter Rückfluß erhitzt. Nach dem Einengen wird der Rückstand in 18.8 ml Methanol aufgenommen und nochmals für 20 Stunden unter Rückfluß erhitzt. Nach dem Einengen wird der so erhaltene Rückstand in 950 ml Toluol gelöst und bei -78 °C unter Argon mit 350 ml einer 1.2 molaren Lösung von Diisobutylaluminiumhydrid versetzt. Man läßt innerhalb von 2 Stunden auf 0 °C erwärmen, gibt dann vorsichtig bei -70 °C 50 ml Isopropanol tropfenweise gefolgt von 175 ml Wasser hinzu und rührt anschließend 2 Stunden bei 24 °C. Man filtriert vom Niederschlag ab, wäscht gut mit Essigsäureethylester nach und dampft im Vakuum ein. Den so erhaltenen Rückstand reinigt man durch Chromatographie an Kieselgel. Mit Hexan / 0-70% Essigsäureethylester erhält man 6.67 g der Titelverbindung als farbloses Öl.
IR (CHCl₃): 3625, 3370 (breit), 3005, 2930, 2862, 1460, 1380, 1075, 1040 cm⁻¹.

### 20b) 5-[cis/trans-1-(tert.-Butyl-dimethylsilyloxymethyl)-cyclohept-2-yl]-(2E,4E)-2,4-pentadiensäureethylester

Zu einer Suspension von 4.1 g Natriumhydrid (55%ig in Mineralöl) in 180 ml Tetrahydrofuran tropft man vorsichtig unter Argon bei 0 °C eine Lösung von 14.9 g des vorstehend hergestellten Diols in 10 ml Tetrahydrofuran. Man rührt 45 Minuten bei 24-°C und gibt dann 14.1 g tert.-Butyl-dimethylsilylchlorid bei 0 °C zu. Man rührt eine Stunde bei 24 °C und verdünnt mit 1000 ml Ether. Man wäscht die organische Phase mit 200 ml 10%iger wässriger Kaliumcarbonat-Lösung und viermal mit je 100 ml gesättigter wässriger Natriumchlorid-Lösung. Nach dem Trocknen über wasserfreiem Magnesiumsulfat und Filtration wird im Vakuum eingeengt. Den so erhaltenen Rückstand reinigt man durch Chromatographie an Kieselgel. Mit Hexan / 0-30% Essigsäureethylester erhält man 22.7 g cis-1-tert.-Butyl-dimethylsilyloxymethyl)-2-hydroxymethyl-cycloheptan als farbloses Öl.
IR (CHCl₃): 3420 (breit), 3008, 2937, 2862, 1470, 1262, 1060, 843 cm⁻¹.

Zu 14.8 g Oxalylchlorid gelöst in 104 ml Dichlormethan tropft man unter Argon vorsichtig bei -60 °C 19.5 g Dimethylsulfoxid gelöst in 42.6 ml Dichlormethan und rührt 10 Minuten bei dieser Temperatur. Anschließend tropft man eine Lösung von 22.7 g des vorstehend hergestellten Alkohols in 42.6 ml Dichlormethan zu und rührt 1 Stunde bei -60 °C. Dann gibt man 26.3 g Triethylamin zu und nach 1 Stunde Rühren bei -60 °C wird die Reaktionsmischung auf 300 ml Eiswasser gegeben. Nach Phasentrennung wird die wäßrige Pase dreimal mit je 300 ml Dichlormethan extrahiert. Die vereinigten organischen Phasen werden mit 100 ml 10%iger wässriger Zitronensäure und anschließend mit halbkonzentrierter wässriger Natriumchlorid-Lösung neutral gewaschen. Nach dem Trocknen über Magnesiumsulfat und Filtration wird im Vakuum eingeengt. Man erhält 22.9 g des Aldehyds, der ohne weitere Reinigung verwendet wird.
IR (CHCl₃): 3005, 2935, 2862, 2740, 1720, 1475, 1258, 1100, 840 cm⁻¹.

Zu einer Suspension von 4.31 g Lithiumchlorid in 430 ml Acetonitril tropft man unter Argon bei 24 °C 36.6 g 4-Phosphonocrotonsäuretriethtylester gefolgt von 12.9 g DBU (Diazabicycloundecan) und rührt für 10 Minuten bei dieser Temperatur. Dann tropft man langsam eine Lösung von 22.9 g des vorstehend hergestellten Aldehyds in 69 ml Acetonitril zu und rührt für 5 Stunden bei 24 °C. Anschließend verdünnt man mit 1.51 l Diethylether, trennt die Phasen, wäscht die organische Phase nacheinander mit 100 ml Wasser und 10%iger wässriger Zitronensäure. Nach dem Neutralwaschen mit halbkonzentrierter wässriger Natriumchlorid-Lösung und Trocknen über wasserfreiem Magnesiumsulfat wird nach Filtration im Vakuum eingeengt. Den so erhaltenen Rückstand reinigt man durch Chromatographie an Kieselgel. Mit Hexan / 0-10% Diethylether erhält man 14.53 g der Titelverbindung als farbloses Öl.
IR (CHCl₃): 30430, 2940, 2870, 1710, 1645, 1622, 1470, 1378, 1310, 1255, 1100, 1008, 843 cm⁻¹.

### 20c) 5-[cis/trans-1-(tert.-Butyl-dimethylsilyloxymethyl)-cyclohept-2-yl]-(2E,4E)-2,4-pentadien-1-al

Zu einer Lösung von 14.5 g des vorstehend hergestellten Esters in 270 ml Toluol tropft man bei -70 °C unter Argon 102 ml einer 1.2 molaren Lösung von Diisobutylaluminiumhydrid in Toluol und läßt innerhalb von 2 Stunden auf 0 °C erwärmen. Man gibt vorsichtig bei -70 °C 5 ml 2-Propanol gefolgt von 51 ml Wasser hinzu und rührt anschließend 2 Stunden bei 24 °C. Man filtriert vom Niederschlag ab, wäscht gut mit Essigsäureethylester nach und engt im Vakuum ein. Den so erhaltenen Rückstand reinigt man durch Chromatographie an Kieselgel. Mit Hexan / 0-10% Essigsäureethylester erhält man 9.22 g 5-[cis/trans-1-(tert.-Butyl-dimethylsilyloxymethyl)-cyclohept-2-yl]-(2E,4E)-2,4-pentadien-1-ol als farbloses Öl.
IR (CHCl₃) : 3420 (breit), 3005, 2940, 2865, 1630, 1465, 1100, 995, 840 cm⁻¹.

Eine Lösung von 9.21 g des vorstehend hergestellten Alkohols in 107 ml Dichlormethan versetzt man mit 19.7 g Braunstein und rührt 18 Stunden bei 24 °C unter Argon. Anschließend wird filtriert und im Vakuum eingeengt. Man erhält 8.57 g der Titelverbindung als farbloses Öl, welches ohne weitere Reinigung verwendet wird.
IR (CHCl₃): 3005, 2935, 2863, 2748, 1680, 1637, 1603, 1467, 1260, 1100, 990, 840 cm⁻¹.

### 20d) (5R oder 5S)-5-Acetoxy-1-[cis-1-hydroxymethyl-cyclohept-2-yl]-(1E,3E)-trideca-1,3-dien (Diastereomer A)

Zu 774 mg Magnesium in 50 ml Tetrahydrofuran tropft man unter Erwärmen eine Lösung aus 6.15 g Octylbromid in 50 ml Tetrahydrofuran und rührt 30 Minuten bei 24 °C. Diese Grignard-Lösung tropft man zu einer Lösung von 8.56 g des unter 1c) hergestellten Aldehyds in 50 ml Tetrahydrofuran bei -30 °C unter Argon zu und rührt 30 Minuten bei dieser Temperatur. Nach Erwärmen auf -10 °C gibt man die Reaktionsmischung auf 500 ml gesättigte wässrige Ammoniumchlorid-Lösung. Man extrahiert dreimal mit je 300 ml Essigsäureethylester und wäscht die vereinigten organischen Phasen anschließend mit gesättigter wässriger Natriumchlorid-Lösung. Nach dem Trocknen über wasserfreiem Magnesiumsulfat und Filtration wird im Vakuum eingeengt. Den so erhaltenen Rückstand reinigt man durch Chromatographie an Kieselgel. Mit Hexan /0-20% Diethylether erhält man 7.40 g (5RS)-5-Hydroxy-1-[cis/trans-1-(tert.-butyl-dimethylsilyloxymethyl)-cyclohept-2-yl]-(1E,3E)-trideca-1,3-dien als farbloses Öl.
IR (CHCl₃): 3700, 3520 (breit), 3005, 2932, 2860, 1465, 1095, 992, 840 cm⁻¹.

Eine Lösung von 7.39 g des vorstehend hergestellten Alkohols in 26.6 ml Pyridin wird mit 13.3 ml Essigsäureanhydrid bei 0 °C unter Argon versetzt und 16 Stunden bei dieser Temperatur gerührt. Anschließend versetzt man mit 50 ml Wasser und engt unter Toluolzugabe im Vakuum ein. Den so erhaltenen Rückstand reinigt man durch Chromatographie an Kieselgel. Mit Hexan / 0-5% Diethylether erhält man 8.1 g (5RS)-5-Acetoxy-1-[cis/trans-1-(tert.-butyl-dimethylsilyloxymethyl)-cyclohept-2-yl]-(1E,3E)-trideca-1,3-dien als farbloses Öl.
IR (CHCl₃): 3005, 2930, 2860, 1725, 1465, 1375, 1253, 1095, 992, 840 cm⁻¹.

Zu einer Lösung von 8.1 g des vorstehend hergestellten Acetats in 77.5 ml Tetrahydrofuran gibt man unter Argon bei 24 °C 12.1 g Tetrabutylammoniumfluoridtrihydrat und rührt drei Stunden bei dieser Temperatur. Anschließend verdünnt man mit 500 ml Diethylether, wäscht dreimal mit je 100 ml Wasser und einmal mit gesättigter wässriger Natriumchlorid-Lösung. Nach dem Trocknen über wasserfreiem Magnesiumsulfat und Filtration wird im Vakuum eingeengt. Den so erhaltenen Rückstand trennt man durch mehrfache Chromatographie an Kieselgel auf. Mit Hexan / Essigsäureethylester (85 + 15) erhält man vier Fraktionen (sortiert nach steigender Polarität):
1. Fraktion: 1.63 g (5R oder 5S)-5-Acetoxy-1-[trans-1-hydroxymethyl-cydohept-2-yl]-(1E,3E)-trideca-1,3-dien (Diastereomer A)
   IR (CHCl₃): 3625, 3460 (breit), 3005, 2935, 2862, 1730, 1468, 1378, 1253, 1020, 995 cm⁻¹.
2. Fraktion: 517 mg der Titelverbindung
   IR (CHCl₃): 3620, 3450 (breit), 3005, 2930, 2860, 1725, 1458, 1375, 1252, 1017, 992 cm⁻¹.
3. Fraktion: 1.36 g (5S oder 5R)-5-Acetoxy-1-[trans-1-hydroxymethyl-cyclohept-2-yl]-(1E,3E)-trideca-1,3-dien (Diastereomer B)
   IR (CHCl₃): 3620, 3450 (breit), 3005, 2930, 2860, 1728, 1465, 1375, 1248, 1017, 990 cm⁻¹.
4. Fraktion: 767 mg (5S oder 5R)-5-Acetoxy-1-[cis-1-hydroxymethyl-cyclohept-2-yl]-(1E,3E)-trideca-1,3-dien (Diastereomer B der Titelverbindung)
   IR (CHCl₃): 3620, 3450 (breit), 3005, 2930, 2860, 1728, 1460, 1375, 1250, 1017, 992 cm⁻¹.

### 20e)cis-(1RS)-1-Formyl-(2RS)-2-((1E,3E)-(5R oder 5S)-5-acetoxy-1,3-tridecadienyl)-cycloheptan (Diastereomer A)

Zu einer Lösung von 501 mg des nach Beispiel 1d) hergestellten Alkohols in 37 ml Dichlormethan gibt man bei 0 °C unter Argon 2.21 g Collins-Reagenz und rührt 80 Minuten bei 0 °C. Dann verdünnt man mit Diethylether und versetzt mit Celite, filtriert über Celite ab und wäscht gut mit Diethylether nach. Nach dem Einengen im Vakuum wird die so erhaltene Titelverbindung ohne weitere Reinigung verwendet.
IR (CHCl₃): 3005, 2935, 2860, 2740, 1725, 1460, 1375, 1250, 990 cm⁻¹.

### Beispiel 21

### (+/-)-(5RS)-5-Acetoxy-5-[cis-(2RS)-2-((1E,3E)-(5R oder 5S)-5-hydroxy-1,3-tridecadienyl-(1RS)-cycloheptyl]-pentansäure (Diastereomer A)

Zu einer Lösung aus 44.3 mg des in Beispiel 1) hergestellten Diacetats in 0.9 ml Methanol gibt man 0.9 ml einer 0.5N wässrige Natronlauge-Lösung und rührt für 2 Stunden bei 24-°C. Anschließend versetzt man mit 20 ml Wasser und stellt einen ph-Wert von 5 mit einer 1N wässrigen Salzsäure ein. Dann wird dreimal mit je 30 ml Essigsäureethylester extrahiert, die vereinigten organischen Phasen werden mit 10 ml gesättigter wässriger Natriumchlorid-Lösung gewaschen, getrocknet über Natriumsulfat und nach Filtration im Vakuum eingeengt. Den so erhaltenen Rückstand reinigt man durch Chromatographie an Kieselgel. Mit Hexan / 0-90% Essigsäureethylester erhält man 34.3 mg der Titelverbindung als farbloses Öl.
IR (Film): 3430 (breit), 3020, 2925, 2855, 1735, 1710, 1456, 1371, 1242, 990 cm⁻¹.

### Beispiel 22

### (+/-)-(5RS)-5-Hydroxy-5-[cis-(2RS)-2-((1E,3E)-(5R oder 5S)-5-hydroxy-1,3-tridecadienyl-(1RS)-cycloheptyl]-pentansäure (Diastereomer A) und (+/-)-(5RS)-5-Hydroxy-5-[cis-(2RS)-2-((1E,3E)-(5R oder 5S)-5-hydroxy-1,3-tridecadienyl-(1RS)-cyclohetyl]-pentansäure-1,5-lacton (Diastereomer A)

Zu einer Lösung aus 68.5 mg des in Beispiel 1) hergestellten Diacetats in 2.85 ml Methanol gibt man 4.3 ml einer 1N wässrige Natronlauge-Lösung und rührt für 18 Stunden bei 24 °C. Anschließend versetzt man mit 30 ml Wasser, wäscht einmal mit Essigsäureethylester und stellt den ph-Wert der wäßrigen Phase mit einer 1N wässriger Salzsäure auf 5 ein. Dann wird dreimal mit je 30 ml Essigsäureethylester extrahiert, die vereinigten organischen Phasen werden mit 20 ml halbkonzentrierter Natriumchlorid-Lösung gewaschen, getrocknet über wasserfreiem Natriumsulfat und nach Filtration im Vakuum eingeengt. Den so erhaltenen Rückstand reinigt man durch Chromatographie an Kieselgel. Mit Hexan / 0-100% Essigsäureethylester erhält man als unpolare Komponente 19.7 mg (+/-)-(5RS)-5-Hydroxy-5-[cis-(2RS)-2-((1E,3E)-(5R oder 5S)-5-hydroxy-1,3-tridecadienyl-(1RS)-cycloheptyl]-pentansäure-1,5-lacton (Diastereomer A) und als polarere Komponente 4.8 mg (+/-)-(5RS)-5-Hydroxy-5-[cis-(2RS)-2-((1E,3E)-(5R oder 5S)-5-hydroxy-1,3-tridecadienyl-(1RS)-cycloheptyl]-pentansäure (Diastereomer A) als farblose öle.
IR (Film; Lacton): 3440 (breit), 3020, 2927, 2855, 1733, 1460, 1377, 1242, 991 cm⁻¹.
IR (CHCl₃; Säure): 3430 (breit), 3005, 2925, 2860, 1725, 1462, 1380, 1240, 990 cm⁻¹.

### Beispiel 23

### (+/-)-(5R oder 5S)-5-Acetoxy-5-[cis-(2RS)-2-((1E,3E)-(5S oder 5R)-5-acetoxy-1,3-tridecadienyl-(1RS)-cycloheptyl]-pentansäure (Diastereomer B, unpol-5)

Zu 303 mg Magnesium tropft man bei 24 °C unter Argon eine Lösung aus 1.39 g 4-Chlor-1-(tert.-butyl-dimethylsilyloxy)-butan in 1.2 ml Tetrahydrofuran, fügt einen Kristall Iod hinzu, erwärmt 10 Minuten auf 70°C, rührt 30 Minuten bei 24 °C und verdünnt mit 3.9 ml Tetrahydrofuran.

Zu einer Lösung von 595 mg cis-(1RS)-1-Formyl-(2RS)-2-[(1E,3E)(5S oder 5R)-5-acetoxy-1,3-tridecadienyl-cycloheptan (Diastereomer B) aus Beispiel 4a) in 3.3 ml Tetrahydrofuran tropft man bei -70°C unter Argon die obig hergestellte Grignard-Lösung, läßt auf -30 °C erwärmen und rührt 1 Stunde bei dieser Temperatur. Man gibt die Reaktionsmischung zu 100 ml gesättigte Ammoniumchlorid-Lösung und extrahiert dreimal mit Essigsäureethylester. Die vereinigten organischen Phasen werden einmal mit halbkonzentrierter wässriger Natriumchlorid-Lösung gewaschen, über wasserfreiem Natriumsulfat getrocknet und nach Filtration im Vakuum eingeengt. Den so erhaltenen Rückstand reinigt man durch mehrfache Chromatographie an Kieselgel. Mit Hexan / Essigsäureethylester (90 + 10) erhält man 131.4 mg des unpolaren diastereomeren Alkohols (unpol-5) und 404.7 mg des polareren diastereomeren Alkohols (pol-5) als farblose Öle.
IR (Film; unpolarer Alkohol): 3650, 3460 (breit), 3022, 2925, 2865, 1734, 1462, 1370, 1248, 1100, 990, 835 cm⁻¹.
IR (Film; polarer Alkohol): 3650, 3460 (breit), 3025, 2930, 2857, 1735, 1237, 1460, 1372, 1100, 990, 836 cm⁻¹.

Zur Acetylierung gibt man zu einer Lösung aus 129 mg des vorstehend beschriebenen unpolaren Alkohols in 0.4 ml Pyridin 0.2 ml Acetanhydrid und rührt 6 Stunden bei 24 °C. Anschließend engt man im Vakuum unter Toluolzusatz ein. Den so erhaltenen Rückstand reinigt man durch Chromatographie an Kieselgel. Mit Hexan / 0-10% Essigsäureethylester erhält man 81.2 mg des Acetats als farbloses Öl.
IR (CHCl₃): 3005, 2930, 2860, 1725, 1465, 1375, 1255, 992, 838 cm⁻¹.

Zur Silyletherspaltung rührt man 77.2 mg des vorstehend hergestellten Acetats in 0.5 ml Tetrahydrofuran mit 91.3 mg Tetrabutylammoniumfluoridtrihydrat bei 24 °C unter Argon für 3 Stunden. Anschließend verdünnt man mit 70 ml Diethylether, wäscht dreimal mit je 20 ml Wasser, einmal mit gesättigter wässriger Natriumchlorid-Lösung, trocknet über wasserfreiem Natriumsulfat und engt nach Filtration im Vakuum ein. Nach Filtration über Kieselgel mit Hexan / Essigsäureethylester erhält man 47.1 mg des 1-Alkohols als farbloses Öl. Dieser wird in 6 ml Essigsäureethylester gelöst und unter Zugabe von 440 mg Platin(IV)oxid in einer Sauerstoffatmosphäre für 4 Stunden gerührt. Anschließend wird vom Katalysator abgesaugt und im Vakuum eingeengt. Den so erhaltenen Rückstand reinigt man durch Chromatographie an Kieselgel. Mit Hexan / 0-50% Essigsäureethylester erhält man 41.3 mg der Titelverbindung als farbloses Öl.
IR (CHCl₃): 3625, 3490 (breit), 3005, 2930, 2860, 1725, 1468, 1373, 1252, 992 cm⁻¹.

Das Ausgangsmaterial für die obige Titelverbindung wird wie folgt hergestellt:

### 23a) cis-(1RS)-1-Formyl-(2RS)-2-((1E,3E)-(5S oder 5R)-5-acetoxy-1,3-tridecadienyl)-cycloheptan (Diastereomer B)

In Analogie zu Beispiel 1e) erhält man aus 753 mg (5S oder 5R)-5-Acetoxy-1-[cis-1-hydroxymethyl-cyclohept-2-yl]-(1E,3E)-trideca-1,3-dien (Diastereomer B, 4. Fraktion aus Beispiel 1d)) 595 mg der Titelverbindung, die ohne weitere Reinigung verwendet wird.
IR (CHCl₃): 3005, 2935, 2858, 2742, 1725, 1460, 1372, 1250, 990 cm⁻¹.

### Beispiel 24

### (+/-)-(5S oder 5R)-5-Acetoxy-5-[cis-(2RS)-2-((1E,3E)-(5S oder 5R)-5-acetoxy-1,3-tridecadienyl-(1RS)-cycloheptyl]-pentansäure (Diastereomer B, pol-5)

Zur Acetylierung gibt man zu einer Lösung aus 400 mg des in Beispiel 23 erhaltenen polareren Alkohols in 1.2 ml Pyridin 0.6 ml Acetanhydrid und rührt 6 Stunden bei 24 °C. Anschließend engt man im Vakuum unter Toluolzusatz ein. Den so erhaltenen Rückstand reinigt man durch Chromatographie an Kieselgel. Mit Hexan / 0-10% Essigsäureethylester erhält man 322.7 mg des Acetats als farbloses Öl.
IR (CHCl₃): 3005, 2930, 2858, 1725, 1464, 1375, 1253, 992, 838 cm⁻¹.

Zur Silyletherspaltung rührt man 318.7 mg des vorstehend hergestellten Acetats in 2.1 ml Tetrahydrofuran mit 376.7 mg Tetrabutylammoniumfluoridtrihydrat bei 24 °C unter Argon für 3 Stunden. Anschließend verdünnt man mit 150 ml Diethylether, wäscht dreimal mit je 30 ml Wasser, einmal mit gesättigter wässriger Natriumchlorid-Lösung, trocknet über wasserfreiem Natriumsulfat und engt nach Filtration im Vakuum ein. Nach Filtration über Kieselgel mit Hexan / Essigsäureethylester erhält man 198 mg des 1-Alkohols als farbloses Öl. Dieser wird in 26 ml Essigsäureethylester gelöst und unter Zugabe von 1.95 g Platin(IV)oxid in einer Sauerstoffatmosphäre für 4 Stunden gerührt. Anschließend wird vom Katalysator abgesaugt und im Vakuum eingeengt. Den so erhaltenen Rückstand reinigt man durch Chromatographie an Kieselgel. Mit Hexan / 0-50% Essigsäureethylester erhält man 200 mg der Titelverbindung als farbloses Öl.
IR (CHCl₃): 3625, 3480 (breit), 3005, 2930, 2860, 1725, 1465, 1373, 1250, 990 cm⁻¹.

### Beispiel 25

### (+/-)-(5R oder 5S)-5-Acetoxy-5-[cis-(2RS)-2-((1E,3E)-(5S oder 5R)-5-hydroxy-1,3-tridecadienyl-(1RS)-cydoheptyl]-pentansäure (Diastereomer B, unpol-5)

In Analogie zu Beispiel 21 erhält man aus 41 mg (+/-)-(5R oder 5S)-5-Acetoxy-5-[cis-(2RS)-2-((1E,3E)-(5S oder 5R)-5-acetoxy-1,3-tridecadienyl-(1RS)-cycloheptyl]-pentansäure (Diastereomer B, unpol-5) aus Beispiel 4) 18.7 mg der Titelverbindung als farbloses Öl.
IR (Film): 3440 (breit), 3020, 2928, 2858, 1737, 1713, 1458, 1375, 1442, 990 cm⁻¹.

### Beispiel 26

### (+/-)-(5S oder 5R)-5-Acetoxy-5-[cis-(2RS)-2-((1E,3E)-(5S oder 5R)-5-hydroxy-1,3-tridecadienyl-(1RS)-cycloheptyl]-pentansäure (Diastereomer B, pol-5)

In Analogie zu Beispiel 21 erhält man aus 80 mg (+/-)-(5S oder 5R)-5-Acetoxy-5-[cis-(2RS)-2-((1E,3E)-(5S oder 5R)-5-acetoxy-1,3-tridecadienyl-(1RS)-cycloheptyl]-pentansäure (Diastereomer B, pol-5) aus Beispiel 5) 62.4 mg der Titelverbindung als farbloses Öl.
IR (Film): 3415, 3100 (breit), 3015, 2920, 2848, 1727, 1695, 1465, 1375, 1255, 990 cm⁻¹.

### Beispiel 27

### (+/-)-(5S oder 5R)-5-Hydroxy-5-[cis-(2RS)-2-((1E,3E)-(5S oder 5R)-5-hydroxy-1,3-tridecadienyl-(1RS)-cycloheptyl]-pentansäure (Diastereomer B, pol-5) und (+/-)-(5S oder 5R)-5-Hydroxy-5-[cis-(2RS)-2-((1E,3E)-(5S oder 5R)-5-hydroxy-1,3-tridecadienyl-(1RS)-cycloheptyl]-pentansäure-1,5-lacton (Diastereomer B, pol-5)

In Analogie zu Beispiel 22 erhält man aus 120 mg (+/-)-(5S oder 5R)-5-Acetoxy-5-[cis-(2RS)-2-((1E,3E)-(5S oder 5R)-5-acetoxy-1,3-tridecadienyl-(1RS)-cycloheptyl]-pentansäure (Diastereomer B, pol-5) aus Beispiel 5) 42.6 mg (+/-)-(5S oder 5R)-5-Hydroxy-5-[cis-(2RS)-2-((1E,3E)-(5S oder 5R)-5-hydroxy-1,3-tridecadienyl-(1RS)-cycloheptyl]-pentansäure-1,5-lacton (Diastereomer B, pol-5) in einer unpolareren Fraktion und 3.5 mg (+/-)(5S oder 5R)-5-Hydroxy-5-[cis-(2RS)-2-((1E,3E)-(5S oder 5R)-5-hydroxy-1,3-tridecadienyl-(1RS)-cycloheptyl]-pentansäure (Diastereomer B, pol-5) in einer polareren Fraktion als farblose Öle.
IR (Film; Lacton): 3445 (breit), 3020, 2928, 2858, 1735, 1460, 1242, 990 cm⁻¹.
IR (CHCl₃; Säure): 3450 (breit), 3005, 2930, 2860, 1730, 1462, 1375, 1250, 990 cm⁻¹.

### Beispiel 28

### (+/-)-(5RS)-5-Acetoxy-5-[trans-(2RS)-2-((1E,3E)-(5R oder 5S)-5-acetoxy-1,3-tridecadienyl-(1RS)-cycloheptyl)-pentan-1-ol (Diastereomer A)

Zu 639 mg Magnesium tropft man bei 24 °C unter Argon eine Lösung aus 2.93 g 4-Chlor-1-(tert.-butyl-dimethylsilyloxy)-butan in 2.5 ml Tetrahydrofuran, fügt einen Kristall Iod hinzu, erwärmt 10 Minuten auf 70 °C, rührt 30 Minuten bei 24 °C und verdünnt mit 8.2 ml Tetrahydrofuran.

Zu einer Lösung von 1.56 g trans-(1RS)-1-Formyl-(2RS)-2-[(1E,3E)-(5R oder 5S)-5-acetoxy-1,3-tridecadienyl-cycloheptan (Diastereomer A) aus Beispiel 9a) in 3 ml Tetrahydrofuran tropft man bei -70 °C unter Argon die obig hergestellte Grignard-Lösung, läßt auf -30 °C erwärmen und rührt 1 Stunde bei dieser Temperatur. Man gibt die Reaktionsmischung auf 100 ml gesättigte wässrige Ammoniumchlorid-Lösung und extrahiert dreimal mit je 100 ml Essigsäureethylester. Die vereinigten organischen Phasen werden einmal mit halbkonzentrierter wässriger Natriumchlorid-Lösung gewaschen, über wasserfreiem Natriumsulfat getrocknet und nach Filtration im Vakuum eingeengt. Den so erhaltenen Rückstand reinigt man durch mehrfache Chromatographie an Kieselgel. Mit Hexan / Essigsäureethylester (85 + 15) erhält man 1.52 g des Alkohols als farbloses Öl.
IR (CHCl₃): 3610, 3460 (breit), 3005, 2930, 2860, 1724, 1467, 1378, 1255, 1100, 994, 840 cm⁻¹.

Zur Acetylierung gibt man zu einer Lösung aus 1.51 g des vorstehend beschriebenen Alkohols in 4.32 ml Pyridin 2.16 ml Acetanhydrid und rührt 18 Stunden bei 0 °C. Anschließend verdünnt man mit 1 ml Wasser und engt im Vakuum unter Toluolzusatz ein. Den so erhaltenen Rückstand reinigt man durch Chromatographie an Kieselgel. Mit Hexan / 0-10% Essigsäureethylester erhält man 1.23 g des Acetats als farbloses Öl.
IR (CHCl₃): 3007, 2938, 2862, 1727, 1468, 1378, 1256, 1100, 993, 842 cm⁻¹.

Zur Silyletherspaltung rührt man 1.23 g des vorstehend hergestellten Acetats in 8.3 ml Tetrahydrofuran mit 1.30 g Tetrabutylammoniumfluoridtrihydrat bei 24 °C unter Argon für 3 Stunden. Anschließend verdünnt man mit 70 ml Diethylether, wäscht dreimal mit je 20 ml Wasser, einmal mit gesättigter wässriger Natriumchlorid-Lösung, trocknet über wasserfreiem Natriumsulfat und engt nach Filtration im Vakuum ein. Den so erhaltenen Rückstand reinigt man durch Chromatographie an Kieselgel. Mit Hexan / 0-30% Essigsäureethylester erhält man 895 mg der Titelverbindung als farbloses Öl.
IR (CHCl₃): 3625, 3460 (breit), 3005, 2930, 2860, 1725, 1460, 1375, 1255, 992 cm⁻¹.

Das Ausgangsmaterial für die obige Titelverbindung wird wie folgt hergestellt:

### 28a)trans-(1RS)-1-Formyl-(2RS)-2-[1E,3E)-(5R oder 5S)-5-acetoxy-1,3-tridecadienylcycloheptan (Diastereomer A)

In Analogie zu Beispiel 20e) erhält man aus 1.61 g (5R oder 5S)-5-Acetoxy-1-[trans-1-hydroxymethyl-cyclohept-2-yl]-(1E,3E)-trideca-1,3-dien (Diastereomer A, 1. Fraktion aus Beispiel 1d)) 1.56 g der Titelverbindung, die ohne weitere Reinigung verwendet wird.
IR (CHCl₃): 3020, 2928, 2858, 2720, 1722, 1460, 1373, 1247, 990 cm⁻¹.

### Beispiel 29

### (+/-)-(5RS)-5-Acetoxy-5-trans-(2RS)-2-((1E,3E)-(5S oder 5R)-5-acetoxy-1,3-tridecadienyl)-(1RS)-cycloheptyl]-pentan-1-ol (Diastereomer B)

Zu 541 mg Magnesium tropft man bei 24 °C unter Argon eine Lösung aus 2.48 g 4-Chlor-1-(tert.-butyl-dimethylsilyloxy)-butan in 2.1 ml Tetrahydrofuran, fügt einen Kristall Iod hinzu, erwärmt 10 Minuten auf 70 °C, rührt 30 Minuten bei 24 °C und verdünnt mit 7.0 ml Tetrahydrofuran.

Zu einer Lösung von 1.31 g trans-(1RS)-1-Formyl-(2RS)-2-[(1E,3E)-(5S oder 5R)-5-acetoxy-1,3-tridecadienyl-cycloheptan (Diastereomer B) aus Beispiel 10a) in 2.5 ml Tetrahydrofuran tropft man bei -70 °C unter Argon die obig hergestellte Grignard-Lösung, läßt auf -30 °C erwärmen und rührt 1 Stunde bei dieser Temperatur. Man gibt die Reaktionsmischung auf 100 ml gesättigte wässrige Ammoniumchlorid-Lösung und extrahiert dreimal mit je 100 ml Essigsäureethylester. Die vereinigten organischen Phasen werden einmal mit halbkonzentrierter wässriger Natriumchlorid-Lösung gewaschen, über wasserfreiem Natriumsulfat getrocknet und nach Filtration im Vakuum eingeengt. Den so erhaltenen Rückstand reinigt man durch mehrfache Flash-Chromatographie an Kieselgel. Mit Hexan / Methyl-tert.-Butylether (92 + 8) erhält man 726 mg des Alkohols als farbloses Öl.
IR (CHCl₃): 3610, 3450 (breit), 3005, 2930, 2860, 1725, 1465, 1375, 1253, 1098, 992, 838 cm⁻¹.

Zur Acetylierung gibt man zu einer Lösung aus 718 mg des vorstehend beschriebenen Alkohols in 2 ml Pyridin 1 ml Acetanhydrid und rührt 18 Stunden bei 0 °C. Anschließend verdünnt man mit 0.5 ml Wasser und engt im Vakuum unter Toluolzusatz ein. Den so erhaltenen Rückstand reinigt man durch Chromatographie an Kieselgel. Mit Hexan / 0-10% Essigsäureethylester erhält man 687 mg des Acetats als farbloses Öl.
IR (CHCl₃): 3005, 2930, 2860, 1726, 1466, 1374, 1253, 1098, 992, 838 cm⁻¹.

Zur Silyletherspaltung rührt man 679 mg des vorstehend hergestellten Acetats in 4.6 ml Tetrahydrofuran mit 723 mg Tetrabutylammoniumfluoridtrihydrat bei 24 °C unter Argon für 3 Stunden. Anschließend verdünnt man mit 50 ml Diethylether, wäscht dreimal mit je 10 ml Wasser, einmal mit gesättigter wässriger Natriumchlorid-Lösung, trocknet über wasserfreiem Natriumsulfat und engt nach Filtration im Vakuum ein. Den so erhaltenen Rückstand reinigt man durch Chromatographie an Kieselgel. Mit Hexan / 0-40% Essigsäureethylester erhält man 552 mg der Titelverbindung als farbloses Öl.
IR (CHCl₃): 3620, 3460 (breit), 3005, 2930, 2860, 1723, 1465, 1375, 1250, 992 cm⁻¹.

Das Ausgangsmaterial für die obige Titelverbindung wird wie folgt hergestellt:

### 29a)trans-(1RS)-1-Formyl-(2RS)-2-[(1E,3E)-(5S oder 5R)-5-acetoxy-1,3-tridecadienylcycloheptan (Diastereomer B)

In Analogie zu Beispiel 20e) erhält man aus 1.36 g (5S oder 5R)-5-Acetoxy-1-[trans-1-hydroxymethyl-cyclohept-2-yl]-(1E,3E)-trideca-1,3-dien (Diastereomer B, 3.Fraktion aus Beispiel 1d)) 1.31 g der Titelverbindung, die ohne weitere Reinigung verwendet wird.
IR (CHCl₃): 3020, 2930, 2858, 2720, 1723, 1460, 1372, 1250, 990 cm⁻¹.

### Beispiel 30

### (+/-)-(5RS)-5-Acetoxy-5-[trans-(2RS)-2-((1E,3E)-(5R oder 5S)-5-acetoxy-1,3-tridecadienyl-(1RS)-cycloheptyl]-pentansäure (Diastereomer A)

Zur Oxidation löst man 891 mg der in Beispiel 28) hergestellten Titelverbindung in 45 ml Dichlormethan, versetzt unter Argon bei 0 °C mit 7.51 g Collins-Reagenz und rührt 1 Stunde bei 0 °C. Anschließend wird mit Diethylether verdünnt, mit Celite versetzt, über Celite abfiltriert und mit Diethylether gut nachgewaschen. Der nach dem Einengen erhaltene 1-Aldehyd wird ohne weitere Reinigung sofort in 51 ml Aceton gelöst, bei - 30 °C unter Argon mit 1.3 ml Jones-Reagenz (J. Chem. Soc. 1953, 2555) versetzt und 20 Minuten bei dieser Temperatur gerührt. Anschließend gibt man 0.5 ml 2-Propanol hinzu, rührt 10 Minuten, verdünnt mit 100 ml Essigsäureethylester, schüttelt viermal mit je 30 ml gesättigte wässrige Natriumchlorid-Lösung, trocknet über wasserfreiem Natriumsulfat und dampft nach Filtration im Vakuum ein. Den so erhaltenen Rückstand reinigt man durch Chromatographie an Kieselgel. Mit Hexan / 0-50% Essigsäureethylester erhält man 483 mg der Titelverbindung als farbloses Öl.
IR (Film): 3450, 3200 (breit),3020, 2930, 2858, 1738, 1710, 1458, 1372, 1242, 990 cm⁻¹.

### Beispiel 31

### (+/-)-(5RS)-5-Acetoxy-5-[trans-(2RS)-2-((1E,3E)-(5S oder 5R)-5-acetoxy-1,3-tridecadienyl)-(1RS)-cycloheptyl]-pentansäure (Diastereomer B)

Zur Oxidation löst man 541 mg der in Beispiel 29) hergestellten Titelverbindung in 27 ml Dichlormethan, versetzt unter Argon bei 0 °C mit 4.57 g Collins-Reagenz und rührt 1 Stunde bei 0 °C. Anschließend wird mit Diethylether verdünnt, mit Celite versetzt, über Celite abfiltriert und mit Diethylether gut nachgewaschen. Der nach dem Einengen erhaltene 1-Aldehyd wird ohne weitere Reinigung sofort in 30 ml Aceton gelöst, bei - 30 °C unter Argon mit 0.75 ml Jones-Reagenz versetzt und 20 Minuten bei dieser Temperatur gerührt. Anschließend gibt man 0.3 ml 2-Propanol hinzu, rührt 10 Minuten, verdünnt mit 100 ml Essigsäureethylester, schüttelt viermal mit je 30 ml halbkonzentzrierte wässrige Natriumchlorid-Lösung, trocknet über wasserfreiem Natriumsulfat und dampft nach Filtration im Vakuum ein. Den so erhaltenen Rückstand reinigt man durch Chromatographie an Kieselgel. Mit Hexan / 0-40% Essigsäureethylester erhält man 326 mg der Titelverbindung als farbloses Öl.
IR (Film): 3450, 3200 (breit), 3020, 2928, 2858, 1738, 1710, 1460, 1372, 1240, 992 cm⁻¹.

### Beispiel 32

### (+/-)-(5RS)-5-Acetoxy-5-[trans-(2RS)-2-((1E,3E)-(5R oder 5S)-5-hydroxy-1,3-tridecadienyl)-(1RS)-cycloheptyl]-pentansäure (Diastereomer A)

In Analogie zu Beispiel 21) erhält man aus 150 mg (+/-)-(5RS)-5-Acetoxy-5-[trans-(2RS)-2-((1E,3E)-(5R oder 5S)-5-acetoxy-1,3-tridecadienyl-(1RS)-cycloheptyl]-pentansäure (Diastereomer A) aus Beispiel 11) 121 mg der Titelverbindung als farbloses Öl.
IR (Film): 3440, 3200 (breit), 3020, 2928, 2857, 1737, 1712, 1458, 1375, 1242, 990 cm⁻¹.

### Beispiel 33

### (+/-)-(5RS)-5-Acetoxy-5-[trans-(2RS)-2-((1E,3E)-(5S oder 5R)-5-hydroxy-1,3-tridecadienyl)-(1RS)-cycloheptyl]-pentansäure (Diastereomer B)

In Analogie zu Beispiel 21) erhält man aus 125 mg (+/-)-(5RS)-5-Acetoxy-5-[trans-(2RS)-2-((1E,3E)-(5S oder 5R)-5-acetoxy-1,3-tridecadienyl-(1RS)-cycloheptyl]-pentansäure (Diastereomer B) aus Beispiel 12) 89.8 mg der Titelverbindung als farbloses Öl.
IR (Film): 3440, 3100 (breit), 3020, 2925, 2860, 1730, 1705, 1467, 1375, 1260, 988 cm⁻¹.

### Beispiel 34

### (+/-)-(5RS)-5-Hydroxy-5-trans-(2RS)-2-((1E,3E)-(5R oder 5S)-5-hydroxy-1,3-tridecadienyl)-(1RS)-cycloheptyl]-pentansäure (Diastereomer A) und (+/-)-(5RS)-5-Hydroxy-5-[trans-(2RS)-2-((1E,3E)-(5R oder 5S)-5-hydroxy-1,3-tridecadienyl-(1RS)-cycloheptyl]-pentansäure-1,5-lacton (Diastereomer A)

In Analogie zu Beispiel 22 erhält man aus 170 mg (+/-)-(5RS)-5-Acetoxy-5-[trans-(2RS)-2-((1E,3E)-(5R oder 5S)-5-acetoxy-1,3-tridecadienyl-(1RS)-cycloheptyl]-pentansäure (Diastereomer A) aus Beispiel 11) 40.1 mg (+/-)-(5RS)-5-Hydroxy-5-[trans-(2RS)-2-((1E,3E)-(5R oder 5S)-5-hydroxy-1,3-tridecadienyl-(1RS)-cycloheptyl]-pentansäure-1,5-lacton (Diastereomer A) in einer unpolareren Fraktion und 8.8 mg (+/-)-(5RS)-5-Hydroxy-5-[trans-(2RS)-2-((1E,3E)-(5R oder 5S)-5-hydroxy-1,3-tridecadienyl-(1RS)-cycloheptyl]-pentansäure (Diastereomer A) in einer polareren Fraktion als farblose Öle.
IR (Film; Lacton): 3440 (breit), 3018, 2930, 2855, 1733, 1465, 1245, 900 cm⁻¹.
IR (Film; Säure): 3435 (breit), 3200 (breit), 3020, 2925, 2855, 1725, 1463, 1380, 1255, 992 cm⁻¹.

### Beispiel 35

### (+/-)-(5RS)-5-Hydroxy-5-[trans-(2RS)-2-((1E,3E)-(5S oder 5R)-5-hydroxy-1,3-tridecadienyl)-(1RS)-cycloheptyl]-pentansäure (Diastereomer B) und (+/-)-(5RS)-5-Hydroxy-5-[trans-(2RS)-2-((1E,3E)-(5S oder 5R)-5-hydroxy-1,3-tridecadienyl-(1RS)-cycloheptyl]-pentansäure-1,5-lacton (Diastereomer B)

In Analogie zu Beispiel 22 erhält man aus 125 mg (+/-)-(5RS)-5-Acetoxy-5-[trans-(2RS)-2-((1E,3E)-(5S oder 5R)-5-acetoxy-1,3-tridecadienyl-(1RS)-cycloheptyl]-pentansäure (Diastereomer B) aus Beispiel 12) 29 mg (+/-)-(5RS)-5-Hydroxy-5-[trans-(2RS)-2-((1E,3E)-(5S oder 5R)-5-hydroxy-1,3-tridecadienyl-(1RS)-cycloheptyl]-pentansäure-1,5-lacton (Diastereomeres B) in einer unpolareren Fraktion und 17.9 mg (+/)-(5RS)-5-Hydroxy-5-[trans-(2RS)-2-((1E,3E)-(5S oder 5R)-5-hydroxy-1,3-tridecadienyl-(1RS)-cycloheptyl]-pentansäure (Diastereomer B) in einer polareren Fraktion als farblose Öle.
IR (Film; Lacton): 3440 (breit), 3020, 2925, 2858, 1732, 1464, 1245, 990 cm⁻¹.
IR (Film; Säure): 3415 (breit), 3015, 2924, 2857, 1713, 1462, 1378, 1250, 990 cm⁻¹.

### Beispiel 36

### (+/-)-(5RS)-5-Acetoxy-5-[trans-(2RS)-2-((1E,3E)-(5R oder 5S)-5-acetoxy-1,3-tridecadienyl)-(1RS)-cycloheptyl]-pentansäuremethylester (Diastereomer A)

Zu einer Lösung von 43 mg (+/-)-(5RS)-5-Acetoxy-5-[trans-(2RS)-2-((1E,3E)-(5R oder 5S)-5-acetoxy-1,3-tridecadienyl-(1RS)-cycloheptyl]-pentansäure (Diastereomer A) aus Beispiel 30) in 4 ml Dichlormethan tropft man bei 0 °C unter Argon eine etherische Diazomethan-Lösung bis zur bleibenden Gelbfärbung, rührt 15 Minuten bei 0 °C und engt anschließend im Vakuum ein. Den so erhaltenen Rückstand reinigt man durch Chromatographie an Kieselgel. Mit Hexan /0-10% Essigsäureethylester erhält man 36 mg der Titelverbindung als farbloses Öl.
IR (CHCl₃): 3005, 2935, 2860, 1730, 1460, 1376, 1255, 990 cm⁻¹.

### Beispiel 37

### (+/-)-(5RS)-5-Hydroxy-5-[trans-(2RS)-2-((1E,3E)-(5S oder 5R)-5-hydroxy-1,3-tridecadienyl)-(1RS)-cycloheptyl]-pentansäure-tris-(hydroxymethyl)-aminomethansalz (Diastereomer B)

Zu einer Lösung von 10 mg (+/-)-(5RS)-5-Hydroxy-5-[trans-(2RS)-2-((1E,3E)-(5S oder 5R)-5-hydroxy-1,3-tridecadienyl-(1RS)-cycloheptyl]-pentansäure (Diastereomer B) aus Beispiel 16) in 1.5 ml Acetonitril gibt man bei 70 °C eine Lösung von 4 mg Tris-(hydroxymethyl)-aminomethan in 0.02 ml Wasser. Man läßt unter Rühren abkühlen, dekantiert nach 16 Stunden vom Lösungsmittel ab und trocknet den Rückstand im Vakuum. Man isoliert 3.3 mg der Titelverbindung als wachsartige Masse.

## Patentansprüche

1. Leukotrien B₄-Derivate der Formel I 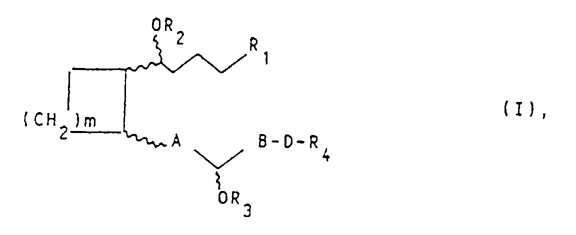 worin
R¹ CH₂OH, CH₃, CF₃, COOR⁵, CONR⁶R⁷ oder ⁷
R¹ gemeinsam mit R² eine Carbonylgruppe bedeutet,
R² und R³ gleich oder verschieden, H oder ein organischer Säurerest mit 1-15 C-Atomen darstellen,
R⁴ H, (C₁-C₁₀) gegebenenfalls einfach oder mehrfach durch Chlor oder Brom substituierter Alkyl, C₃-C₁₀ Cycloalkyl, gegebenenfalls unabhängig voneinander einfach oder mehrfach durch Chlor, Brom. Phenyl C₁-C₄-Alkyl, C₁-C₄-Alkoxy, Fluormethyl, Chlormethyl, Trifluormethyl, Carboxy oder Hydroxy substituiertes C₆-C₁₀ Arylrest oder einen 5-6 gliedriger aromatischer heterocyclischen Ring mit wenigstens 1 Heteroatom symbolisieren,
R⁵ Wasserstoff, C₁-C₁₀ Alkyl, C₃-C₁₀ Cycloalkyl, gegebenenfalls durch 1-3 Chlor, Brom, Phenyl, C₁-C₄-Alkyl, C₁-C₄-Alkoxy, Fluormethyl, Chlormethyl, Trifluormethyl, Carboxy oder Hydroxy substituierter C₆-C₁₀ Arylrest, CH₂-CO-(C₆-C₁₀) Aryl oder ein 5-6 gliedriger Ring mit wenigstens 1 Heteroatom bedeutet,
A eine trans,trans-CH=CH-CH=CH, eine -CH₂CH₂-CH=CH- oder eine Tetramethylengruppe darstellt,
B eine C₁-C₁₀ grad- oder verzweigtkettige Alkylengruppe, die gegebenenfalls durch Fluor substituiert sein kann oder die Gruppe 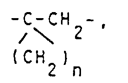 symbolisiert,
D eine Direktbindung, Sauerstoff, Schwefel, -C≡C-,-CH=CR⁸, oder gemeinsam mit
B auch eine Direktbindung bedeuten können,
R⁶ und R⁷ gleich oder verschieden sind und H oder C₁-C₄ Alkyl oder R⁷ H und R⁶ C₁-C₁₀-Alkanoyl oder C₁-C₁₀ Alkansulfonyl darstellen.
R⁸ H, C₁-C₅ Alkyl, Chlor, Brom bedeutet,
m die Ziffern 1 oder 4 symbolisiert und
n 3-5 ist sowie, wenn R⁵ Wasserstoff bedeutet, deren Salze mit physiologisch verträglichen Basen und deren Cyclodextrinclathrate.

2. (+/-)-(5RS)-5-Acetoxy-5-[cis-(2RS)-2-((1E,3E)-(5RS)-5-acetoxy-1,3-tridecadienyl)-(1RS)-cyclobutyl]-pentan-1-ol.

3. (+/-)-(5RS)-5-Acetoxy-5-[cis-(2RS)-2-((1E,3E)-(5RS)-5-acetoxy-1,3-tridecadienyl)-(1RS)-cyclobutyl)-pentansäure.

4. (+/-)-(5RS)-5-Hydroxy-5-[cis-(2RS)-2-((1E,3E)-(5RS)-5-hydroxy-1,3-tridecadienyl)-(1RS)-cycloheptyl]-pentansäure.

5. (+/-)-(5RS)-5-Acetoxy-5-[cis-(2RS)-2-((1E,3E)-(5RS)-5-hydroxy-1,3-tridecadienyl)-(1RS)-cyclobutyl]-pentansäure.

6. (+/-)-(5RS)-5-Hydroxy-5-[cis-(2RS)-2-((1E,3E)-(5RS)-5-hydroxy-1,3-tridecadienyl)-(1RS)-cyclobutyl]-pentan-1-ol.

7. (+/-)-(5RS)-5-Acetoxy-5-[trans-(2RS)-2-((1E,3E)-(5RS)-5-acetoxy-1,3-tridecadienyl)-(1RS)-cyclobutyl]-pentansäure.

8. (+/-)-(5RS)-5-Acetoxy-5-[trans-(2RS)-2-((1E,3E)-(5RS)-5-acetoxy-1,3-tridecadienyl)-(1RS)-cyclobutyl]-pentan-1-ol.

9. (+/-)-(5RS)-5-hydroxy-5-[trans-(2RS)-2-((1E,3E)-(5RS)-5-hydroxy-1,3-tridecadienyl)-(1RS)-cyclobutyl]-pentansäure.

10. (+/-)-(5RS)-5-Acetoxy-5-[trans-(2RS)-2-((1E,3E)-(5RS)-5-hydroxy-1,3-tridecadienyl)-(1RS)-cyclobutyl]-pentansäure.

11. (+/-)-(5RS)-5-Hydroxy-5-[cis-(2RS)-2-((1E,3E)-(5RS)-5-hydroxy-1,3-tridecadienyl)-(1RS)-cyclobutyl]-pentansäuremethylester.

12. (+/-)-(5RS)-5-Acetoxy-5-[cis-(2RS)-2-((1E,3E)-(5RS)-5hydroxy-1,3-tridecadienyl)-(1RS)-cyclobutyl]-pentansäuremethylester.

13. (+/-)-(5RS)-5-hydroxy-5-[cis-(2RS)-2-((1E,3E)-(5RS)-5-hydroxy-1,3-tridecadienyl)-(1RS)-cyclobutyl]-pentansäure-tris-(hydroxymethyl)-aminomethansalz.

14. (+/-)-(5RS)-5-hydroxy-5-[cis-(2RS)-2-((1E,3E)-(5RS)-5-hydroxy-1,3-tridécadiényl)-(1RS)-cyclobutyl]-pentansäure-1,5-lacton.

15. Pharmazeutische Präparate, gekennzeichnet durch eine Gehalt an ein oder zwei Leukotrien-B₄-Derivate gemäß Anspruch 1 bis 14.

16. (+/-)-(5RS)-5-Acetoxy-5-[cis-(2RS)-2-((1E,3E)-(5RS)-5-acetoxy-1,3-tridecadienyl)-(1RS)-cyclobutyl]-pentan-1-ol.

17. (+/-)-(5RS)-5-Acetoxy-5-[cis-(2RS)-2-((1E,3E)-(5RS)-5-acetoxy-1,3-tridecadienyl)-(1RS)-cyclobutyl]-pentansäure.

18. (+/-)-(5RS)-5-hydroxy-5-[cis-(2RS)-2-((1E,3E)-(5RS)-5-hydroxy-1,3-tridecadienyl)-(1RS)-cycloheptyl]-pentansäure.

19. (+/-)-(5RS)-5-Acetoxy-5-[cis-(2RS)-2-((1E,3E)-(5RS)-5-hydroxy-1,3-tridecadienyl)-(1RS)-cyclobutyl]-pentansäure.

20. (+/-)-(5RS)-5-hydroxy-5-[cis-(2RS)-2-((1E,3E)-(5RS)-5-hydroxy-1,3-tridecadienyl)-(1RS)-cyclobutyl]-pentan-1-ol.

21. (+/-)-(5RS)-5-Acetoxy-5-[trans-(2RS)-2-((1E,3E)-(5RS)-5-acetoxy-1,3-tridecadienyl)-(1RS)-cycloheptyl]-pentan-1-ol.

22. (+/-)-(5RS)-5-acetoxy-5-[trans-(2RS)-2-((1E,3E)-(5RS)-5-acetoxy-1,3-tridecadienyl)-(1RS)-cycloheptyl]-pentansäure.

23. (+/-)-(5RS)-5-Acetoxy-5-[trans-(2RS)-2-((1E,3E)-(5RS)-5-hydroxy-1,3-tridecadienyl)-(1RS)-cycloheptyl]-pentansäure.

24. (+/-)-(5RS)-5-hydroxy-5-[trans-(2RS)-2-((1E,3E)-(5RS)-5-hydroxy-1,3-tridecadienyl)-(1RS)-cycloheptyl]-pentansäure.

25. (+/-)-(5RS)-5-hydroxy-5-[trans-(2RS)-2-((1E,3E)-(5RS)-5-hydroxy-1,3-tridecadienyl)-(1RS)-cycloheptyl]-pentansäure-1,5-lacton.

26. (+/-)-(5RS)-5-Acetoxy-5-[trans-(2RS)-2-((1E,3E)-(5RS)-5-acetoxy-1,3-tridecadienyl)-(1RS)-cycloheptyl]-pentansäure-methylester.

27. (+/-)-(5RS)-5-hydroxy-5-[trans-(2RS)-2-((1E,3E)-(5RS)-5-hydroxy-1,3-tridecadienyl)-(1RS)-cycloheptyl]-pentansäure-tris-(hydroxymethyl)-aminomethansalz.

28. Pharmazeutische Präparate, gekennzeichnet durch ein oder zwei Leukotrien-B₄-Derivate gemäß Anspruch 1 bis 28.

29. Verfahren zur Herstellung von Leukotrien-B₄-Derivaten der Formel I, gemäß Patentanspruch 1, dadurch gekennzeichnet, daß man einen Aldehyd der Formel II 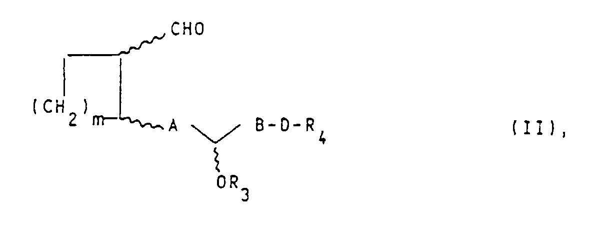 worin m, A, B, D, R³ und R⁴ die oben angegebene Bedeutung haben, gegebenenfalls nach Schutz freier Hydroxygruppen mit einer magnesiumorganischen Verbindung der Formel III,
X-Mg-CH₂-CH₂-CH₂-CH₂-R⁹ (III),
worin X Chlor, Brom oder Iod und R⁹ -CH₃, CF₃ oder -OR¹⁰ darstellt, worin R₁₀ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹=CONHR⁶R⁷) überführt oder eine Carboxylgruppe mit einer physiologisch verträglichen Base in ein Salz überführt.

## Claims

1. Leucotriene B₄ derivatives of the formula I 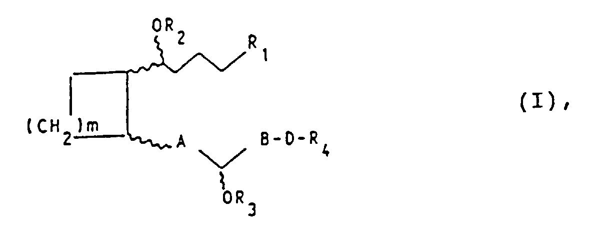 wherein
R¹ represents CH₂OH, CH₃, CF₃, COOR⁵, CONR⁶R⁷ or
R¹ together with R² represents a carbonyl group,
R² and R³ are identical or different and represent H or an organic acid radical having 1-15 carbon atoms,
R⁴ represents H, (C₁-C₁₀) alkyl optionally mono- or polysubstituted by chlorine or bromine; C₃-C₁₀ cycloalkyl; a C₆-C₁₀ aryl radical optionally mono- or polysubstituted by chlorine, bromine, phenyl, C₁-C₄-alkyl, C₁-C₄-alkoxy, fluoromethyl, chloromethyl, trifluoromethyl, carboxy or hydroxy independently of one another; or a 5- or 6-membered aromatic heterocyclic ring having at least 1 hetero atom,
R⁵ represents hydrogen, C₁-C₁₀ alkyl, C₃-C₁₀ cycloalkyl, a C₆-C₁₀ aryl radical optionally substituted by 1-3 chlorine, bromine, phenyl, C₁-C₄-alkyl, C₁-C₄-alkoxy, fluoromethyl, chloromethyl, trifluoromethyl, carboxy or hydroxy substituent(s), CH₂-CO-(C₆-C₁₀) aryl or a 5- or 6-membered ring having at least 1 hetero atom,
A represents a trans,trans-CH=CH-CH=CH-, a -CH₂CH₂-CH=CH- or a tetramethylene group,
B represents a straight-chain or branched-chain alkylene group that may optionally be substituted by fluorine or represents the group 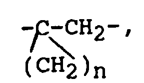
D may represent a direct bond, oxygen, sulphur, -C≡C-, -CH=CR⁸-, or together with
B also a direct bond,
R⁶ and R⁷ are identical or different and represent H or C₁-C₄ alkyl or R⁷ represents H and R⁶ represents C₁-C₁₀-alkanoyl or C₁-C₁₀-alkanesulphonyl,
R⁸ represents H, C₁-C₅ alkyl, chlorine, bromine,
m represents the numbers 1 or 4 and
n is 3-5,
and, when R⁵ represents hydrogen, the salts thereof with physiologically tolerable bases and the cyclodextrin clathrates thereof.

2. (+/-)-(5RS)-5-acetoxy-5-[cis-(2RS)-2-((1E,3E)-(5RS)-5-acetoxy-1,3-tridecadienyl)-(1RS)-cyclobutyl]-pentan-1-ol.

3. (+/-)-(5RS)-5-acetoxy-5-[cis-(2RS)-2-((1E,3E)-(5RS)-5-acetoxy-1,3-tridecadienyl)-(1RS)-cyclobutyl]-pentanoic acid.

4. (+/-)-(5RS)-5-hydroxy-5-[cis-(2RS)-2-((1E,3E)-(5RS)-5-hydroxy-1,3-tridecadienyl)-(1RS)-cycloheptyl]-pentanoic acid.

5. (+/-)-(5RS)-5-acetoxy-5-[cis-(2RS)-2-((1E,3E)-(5RS)-5-hydroxy-1,3-tridecadienyl)-(1RS)-cyclobutyl]-pentanoic acid.

6. (+/-)-(5RS)-5-hydroxy-5-[cis-(2RS)-2-((1E,3E)-(5RS)-5-hydroxy-1,3-tridecadienyl)-(1RS)-cyclobutyl]-pentan-1-ol.

7. (+/-)-(5RS)-5-acetoxy-5-[trans-(2RS)-2-((1E,3E)-(5RS)-5-acetoxy-1,3-tridecadienyl)-(1RS)-cyclobutyl]-pentanoic acid.

8. (+/-)-(5RS)-5-acetoxy-5-[trans-(2RS)-2-((1E,3E)-(5RS)-5-acetoxy-1,3-tridecadienyl)-(1RS)-cyclobutyl]-pentan-1-ol.

9. (+/-)-(5RS)-5-hydroxy-5-[trans-(2RS)-2-((1E,3E)-(5RS)-5-hydroxy-1,3-tridecadienyl)-(1RS)-cyclobutyl]-pentanoic acid.

10. (+/-)-(5RS)-5-acetoxy-5-[trans-(2RS)-2-((1E,3E)-(5RS)-5-hydroxy-1,3-tridecadienyl)-(1RS)-cyclobutyl]-pentanoic acid.

11. (+/-)-(5RS)-5-hydroxy-5-[cis-(2RS)-2-((1E,3E)-(5RS)-5-hydroxy-1,3-tridecadienyl)-(1RS)-cyclobutyl]-pentanoic acid methyl ester.

12. (+/-)-(5RS)-5-acetoxy-5-[cis-(2RS)-2-((1E,3E)-(5RS)-5-hydroxy-1,3-tridecadienyl)-(1RS)-cyclobutyl]-pentanoic acid methyl ester.

13. (+/-)-(5RS)-5-hydroxy-5-[cis-(2RS)-2-((1E,3E)-(5RS)-5-hydroxy-1,3-tridecadienyl)-(1RS)-cyclobutyl]-pentanoic acid tris-(hydroxymethyl)-aminomethane salt.

14. (+/-)-(5RS)-5-hydroxy-5-[cis-(2RS)-2-((1E,3E)-(5RS)-5-hydroxy-1,3-tridecadienyl)-(1RS)-cyclobutyl]-pentanoic acid 1,5-lactone.

15. Pharmaceutical preparations, characterised by a content of one or two leucotriene B₄ derivatives according to claims 1 to 14.

16. (+/-)-(5RS)-5-acetoxy-5-[cis-(2RS)-2-((1E,3E)-(5RS)-5-acetoxy-1,3-tridecadienyl)-(1RS)-cyclobutyl]-pentan-1-ol.

17. (+/-)-(5RS)-5-acetoxy-5-[cis-(2RS)-2-((1E,3E)-(5RS)-5-acetoxy-1,3-tridecadienyl)-(1RS)-cyclobutyl]-pentanoic acid.

18. (+/-)-(5RS)-5-hydroxy-5-[cis-(2RS)-2-((1E,3E)-(5RS)-5-hydroxy-1,3-tridecadienyl)-(1RS)-cycloheptyl]-pentanoic acid.

19. (+/-)-(5RS)-5-acetoxy-5-[cis-(2RS)-2-((1E,3E)-(5RS)-5-hydroxy-1,3-tridecadienyl)-(1RS)-cyclobutyl]-pentanoic acid.

20. (+/-)-(5RS)-5-hydroxy-5-[cis-(2RS)-2-((1E,3E)-(5RS)-5-hydroxy-1,3-tridecadienyl)-(1RS)-cyclobutyl]-pentan-1-ol.

21. (+/-)-(5RS)-5-acetoxy-5-[trans-(2RS)-2-((1E,3E)-(5RS)-5-acetoxy-1,3-tridecadienyl)-(1RS)-cycloheptyl]-pentan-1-ol.

22. (+/-)-(5RS)-5-acetoxy-5-[trans-(2RS)-2-((1E,3E)-(5RS)-5-acetoxy-1,3-tridecadienyl)-(1RS)-cycloheptyl]-pentanoic acid.

23. (+/-)-(5RS)-5-acetoxy-5-[trans-(2RS)-2-((1E,3E)-(5RS)-5-hydroxy-1,3-tridecadienyl)-(1RS)-cycloheptyl]-pentanoic acid.

24. (+/-)-(5RS)-5-hydroxy-5-[trans-(2RS)-2-((1E,3E)-(5RS)-5-hydroxy-1,3-tridecadienyl)-(1RS)-cycloheptyl]-pentanoic acid.

25. (+/-)-(5RS)-5-hydroxy-5-[trans-(2RS)-2-((1E,3E)-(5RS)-5-hydroxy-1,3-tridecadienyl)-(1RS)-cycloheptyl]-pentanoic acid 1,5-lactone.

26. (+/-)-(5RS)-5-acetoxy-5-[trans-(2RS)-2-((1E,3E)-(5RS)-5-acetoxy-1,3-tridecadienyl)-(1RS)-cycloheptyl]-pentanoic acid methyl ester.

27. (+/-)-(5RS)-5-hydroxy-5-[trans-(2RS)-2-((1E,3E)-(5RS)-5-hydroxy-1,3-tridecadienyl)-(1RS)-cycloheptyl]-pentanoic acid tris-(hydroxymethyl)-aminomethane salt.

28. Pharmaceutical preparations, characterised by one or two leucotriene B₄ derivatives according to claims 1 to 28.

29. Process for the manufacture of leucotriene B₄ derivatives of the formula I according to patent claim 1, characterised in that an aldehyde of the formula II 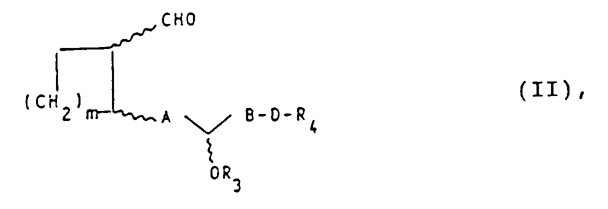 wherein m, A, B, D, R³ and R⁴ have the meanings given above, is reacted, optionally after protection of free hydroxy groups, with an organomagnesium compound of the formula III
X-Mg-CH₂-CH₂-CH₂-CH₂-R⁹ (III),
wherein X represents chlorine, bromine or iodine and R⁹ represents -CH₃, CF₃ or -OR¹⁰ wherein R₁₀ represents a readily removable ether radical and, optionally, subsequently in any desired sequence isomers are separated, protected hydroxy groups are freed and/or a free hydroxy group is esterified and/or the 1-hydroxy group is oxidised to the carboxylic acid and/or double bonds are hydrogenated and/or an esterified carboxy group (R¹ = COOR⁵) is hydrolysed and/or reduced and/or a carboxy group (R⁵ = H) is esterified and/or a free carboxy group (R⁵ = H) is converted into an amide (R¹ = CONHR⁶R⁷), or a carboxy group is converted into a salt with a physiologically tolerable base.

## Revendications

1. Dérivés de leucotriènes-B₄ de formule I : 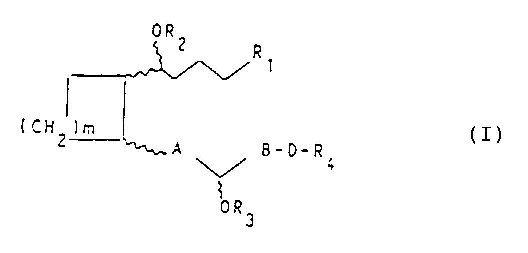 dans laquelle
R¹ représente CH₂OH, CH₃, CF₃, COOR⁵, CONR⁶R⁷, ou
R¹ ensemble avec R² représente un groupe carbonyle,
R² et R³ sont identiques ou différents et représentent H ou un radical d'acide organique avec 1 à 15 atomes de carbone,
R⁴ représente H, alkyle en C₁-C₁₀, éventuellement substitué une ou plusieurs fois par le chlore ou le brome, cycloalkyle en C₃-C₁₀, un radical aryle en C₆-C₁₀ éventuellement substitués indépendamment les uns des autres une ou plusieurs fois par le chlore, le brome, phényle, alkyle en C₁-C₄, alcoxy en C₁-C₄, fluorométhyle, chlorométhyle, trifluorométhyle, carboxyle ou hydroxy ou représente un cycle hétérocyclique aromatique à 5 à 6 chaînons avec au moins un hétéroatome,
R⁵ représente l'hydrogène, alkyle en C₁-C₁₀, cycloalkyle en C₃-C₁₀, radical aryle en C₆-C₁₀ éventuellement substitué par 1 à 3 chlore, brome, phényle, alkyle en C₁-C₄, alcoxy en C₁-C₄, fluorométhyle, chlorométhyle, trifluorométhyle, carboxyle ou hydroxy, CH₂-CO-(C₆-C₁₀)-aryle ou un cycle à 5 à 6 chaînons avec au moins un hétéroatome,
A représente un groupe trans,trans-CH=CH-CH=CH, un CH₂- CH₂-CH=Ch- ou un groupe tétraméthylène,
B représente un groupe alkylène en C₁-C₁₀ linéaire ou ramifié, qui peut être éventuellement substitué par fluor ou le groupe 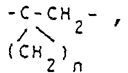
D représente une liaison directe, l'oxygène, le soufre, -C≡C-, -CH=CR⁸ ou ensemble avec
B peut aussi signifier une liaison directe,
R⁶ et R⁷ sont identiques ou différents et représentent H ou alkyle en C₁ -C₄ ou R⁷ représente H et R⁶ représente alcanoyle en C₁-C₁₀ ou (alcane en C₁-C₁₀)-sulfonyle,
R⁸ représente H, alkyle en C₁-C₅, le chlore, le brome,
m vaut 1 ou 4 et
n va de 3 à 5 ainsi que lorsque
R⁵ représente l'hydrogène, ses sels avec des bases physiologiquement compatibles ou leurs clathrates avec la cyclodextrine.

2. (+/-)-(5RS)-5-Acétoxy-5-[cis-(2RS)-2-((1E,3E)-(5RS)-5-acétoxy-1,3-tridécadiényl)-(1RS)-cyclobutyl]-pentan-1-ol.

3. Acide (+/-)-(5RS)-5-acétoxy-5-[cis-(2RS)-2-((1E,3E)-(5RS)-5-acétoxy-1,3-tridécadiényl)-(1RS)-cyclobutyl]-pentanoïque.

4. Acide (+/-)-(5RS)-5-hydroxy-5-[cis-(2RS)-2-((1E,3E)-(5RS)-5-hydroxy-1,3-tridécadiényl)-(1RS)-cycloheptyl]-pentanoïque.

5. Acide (+/-)-(5RS)-5-acétoxy-5-[cis-(2RS)-2-((1E,3E)-(5RS)-5-hydroxy-1,3-tridécadiényl)-(1RS)-cyclobutyl]-pentanoïque.

6. (+/-)-(5RS)-5-Hydroxy-5-[cis-(2RS)-2-((1E,3E)-(5RS)-5-hydroxy-1,3-tridécadiényl)-(1RS)-cyclobutyl]-pentan-1-ol.

7. Acide (+/-)-(5RS)-5-acétoxy-5-[trans-(2RS)-2-((1E,3E)-(5RS)-5-acétoxy-1,3-tridécadiényl)-(1RS)-cyclobutyl]-pentanoïque.

8. (+/-)-(5RS)-5-Acétoxy-5-[trans-(2RS)-2-((1E,3E)-(5RS)-5-acétoxy-1,3-tridécadiényl)-(1RS)-cyclobutyl]-pentan-1-ol.

9. Acide (+/-)-(5RS)-5-hydroxy-5-[trans-(2RS)-2-((1E,3E)-(5RS)-5-hydroxy-1,3-tridécadiényl)-(1RS)-cyclobutyl]-pentanoïque.

10. Acide (+/-)-(5RS)-5-acétoxy-5-[trans-(2RS)-2-((1E,3E)-(5RS)-5-hydroxy-1,3-tridécadiényl)-(1RS)-cyclobutyl]-pentanoïque.

11. (+/-)-(5RS)-5-Hydroxy-5-[cis-(2RS)-2-((1E,3E)-(5RS)-5-hydroxy-1,3-tridécadiényl)-(1RS)-cyclobutyl]-pentanoate de méthyle.

12. (+/-)-(5RS)-5-Acétoxy-5-[cis-(2RS)-2-((1E,3E)-(5RS)-5hydroxy-1,3-tridécadiényl)-(1RS)-cyclobutyl]-pentanoate de méthyle.

13. Sel tris-(hydroxyméthyl)aminométhanoïque d'acide (+/-)-(5RS)-5-hydroxy-5-[cis-(2RS)-2-((1E,3E)-(5RS)-5-hydroxy-1,3-tridécadiényl)-(1RS)-cyclobutyl]-pentanoïque.

14. (+/-)-(5RS)-5-hydroxy-5-[cis-(2RS)-2-((1E,3E)-(5RS)-5-hydroxy-1,3-tridécadiényl)-(1RS)-cyclobutyl]-valéro-1,5-lactone.

15. Préparations pharmaceutiques caractérisées par une teneur en un ou deux dérivés de leucotriène-B₄ selon les revendications 1 à 14.

16. (+/-)-(5RS)-5-Acétoxy-5-[cis-(2RS)-2-((1E,3E)-(5RS)-5-acétoxy-1,3-tridécadiényl)-(1RS)-cyclobutyl]-pentan-1-ol.

17. Acide (+/-)-(5RS)-5-acétoxy-5-[cis-(2RS)-2-((1E,3E)-(5RS)-5-acétoxy-1,3-tridécadiényl)-(1RS)-cyclobutyl]-pentanoïque.

18. Acide (+/-)-(5RS)-5-hydroxy-5-[cis-(2RS)-2-((1E,3E)-(5RS)-5-hydroxy-1,3-tridécadiényl)-(1RS)-cycloheptyl]-pentanoïque.

19. Acide (+/-)-(5RS)-5-acétoxy-5-[cis-(2RS)-2-((1E,3E)-(5RS)-5-hydroxy-1,3-tridécadiényl)-(1RS)-cyclobutyl]-pentanoïque.

20. (+/-)-(5RS)-5-Hydroxy-5-[cis-(2RS)-2-((1E,3E)-(5RS)-5-hydroxy-1,3-tridécadiényl)-(1RS)-cyclobutyl]-pentan-1-ol.

21. (+/-)-(5RS)-5-Acétoxy-5-[trans-(2RS)-2-((1E,3E)-(5RS)-5-acétoxy-1,3-tridécadiényl)-(1RS)-cycloheptyl]-pentan-1-ol.

22. Acide (+/-)-(5RS)-5-acétoxy-5-[trans-(2RS)-2-((1E,3E)-(5RS)-5-acétoxy-1,3-tridécadiényl)-(1RS)-cycloheptyl]-pentanoïque.

23. Acide (+/-)-(5RS)-5-acétoxy-5-[trans-(2RS)-2-((1E,3E)-(5RS)-5-hydroxy-1,3-tridécadiényl)-(1RS)-cycloheptyl]-pentanoïque.

24. Acide (+/-)-(5RS)-5-hydroxy-5-[trans-(2RS)-2-((1E,3E)-(5RS)-5-hydroxy-1,3-tridécadiényl)-(1RS)-cycloheptyl]-pentanoïque.

25. (+/-)-(5RS)-5-hydroxy-5-[trans-(2RS)-2-((1E,3E)-(5RS)-5-hydroxy-1,3-tridécadiény1)-(1RS)-cycloheptyl]-valéro-1,5-lactone.

26. (+/-)-(5RS)-5-Acétoxy-5-[trans-(2RS)-2-((1E,3E)-(5RS)-5-acétoxy-1,3-tridécadiényl)-(1RS)-cycloheptyl]-pentanoate de méthyle.

27. Sel tris-(hydroxyméthyl)-aminométhanoïque d'acide (+/-)-(5RS)-5-hydroxy-5-[trans-(2RS)-2-((1E,3E)-(5RS)-5-hydroxy-1,3-tridécadiényl)-(1RS)-cycloheptyl]-pentanoïque.

28. Préparations pharmaceutiques caractérisées par une teneur en un ou deux dérivés de leucotriène-B₄ selon les revendications 1 à 28.

29. Procédé pour la préparation de dérivés de leucotriène-B₄ de formule I selon la revendication 1, caractérisé en ce que l'on fait réagir un aldéhyde de formule II : 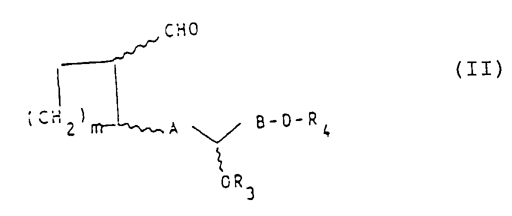 dans laquelle m, A, B, R³ et R⁴ ont la signification donnée ci-dessus, éventuellement après protection des groupes hydroxy libres par un composé organomagnésium de formule III :
X-Mg-CH₂-CH₂-CH₂-R⁹ (III)
dans laquelle X représente le chlore, le brome ou l'iode et R⁹ représente -CH₃, CF₃ ou -OR¹⁰, dans laquelle R¹⁰ représente un radical éther facilement dissociable, et éventuellement ensuite on sépare les isomères dans une succession d'opérations quelconques, on libère les groupes hydroxy protégés et/ou on estérifie un groupe hydroxy libre et/ou on oxyde le groupe 1-hydroxy en acide carboxylique et/ou on hydrogène les doubles liaisons et/ou on saponifie un groupe carboxyle estérifié (R¹=COOR⁵) et/ou on réduit et/ou on estérifie un groupe carboxyle (R⁵=H) et/ou on transforme un groupe carboxyle libre (R⁵=H) en un amide (R¹= CONHR⁶R⁷) ou on transforme un groupe carboxyle avec une base physiologiquement compatible en un sel.
